(19) Europäisches Patentamt
European Patent Office
Office européen des brevets

(11) EP 2 924 039 A1

(12) **EUROPEAN PATENT APPLICATION**

(43) Date of publication:
**30.09.2015 Bulletin 2015/40**

(51) Int Cl.:
***C07D 487/04*** *(2006.01)* ***A61K 31/497*** *(2006.01)*
***A61P 35/00*** *(2006.01)*

(21) Application number: **14162145.8**

(22) Date of filing: **27.03.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universität Zürich**
**8006 Zürich (CH)**

(72) Inventors:
- **Unzue Lopez, Andrea**
  **8050 Zurich (CH)**
- **Dong, Jing**
  **8051 Zurich (CH)**
- **Lafleur, Karine Anne**
  **4310 Rheinfelden (CH)**
- **Zhao, Hongtao**
  **8044 Zurich (CH)**
- **Frugier, Emilie**
  **5400 Baden (CH)**
- **Caflisch, Amedeo**
  **8057 Zurich (CH)**
- **Nevado Blazquez, Cristina**
  **8057 Zurich (CH)**

(74) Representative: **Bolsinger, Jens Roland**
**Schulz Junghans**
**Patentanwälte PartGmbB**
**Großbeerenstraße 71**
**10963 Berlin (DE)**

# (54) 2-Amino-1-phenyl-pyrrolo[3,2-b]quinoxaline-3-carboxamide derivates

(57) The invention relates to compound characterized by a general formula (1),

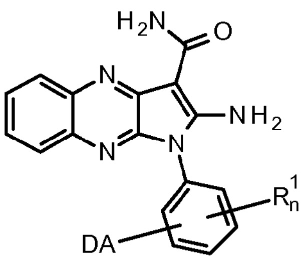

(1)

wherein n of $R^1_n$ is 0, 1, 2, 3 or 4, in particular n of $R^1_n$ is 0 or 1, and each $R^1$ independently from any other $R^1$ is $C_1$-$C_3$ alkyl, $C_1$-$C_3$ haloalkyl or $C_1$-$C_3$ alkoxy, and DA is a Donor-Acceptor group. The invention relates further to a compound or a pharmaceutical preparation for use in a method for treatment of cancer or intraocular neovascular syndromes.

EP 2 924 039 A1

## Description

### Field of the invention

The invention relates to 2-amino-1-phenyl-pyrrolo[3,2-b]quinoxaline-3-carboxamide derivates, pharmaceutical preparations thereof and their use in the treatment of cancer and intraocular neovascular syndromes.

### Background of the invention

Receptor tyrosine kinases are attractive targets for cancer therapeutics. For instance, the receptor tyrosine kinase EphB4 is involved in many types of cancer (Cheng N., Brantley D.M. and Chen J., Cytokine Growth Factor Rev., 2002, 13:75-85).

Ephrins and their receptors were first identified in studies for neuronal growth during development. Unlike other families of receptor tyrosine kinases, which bind soluble ligands, ephrin receptors interact with cell surface-bound ephrin ligands. Ephrins attach to the cell membrane either through a glycosylphosphatidyl inositol anchor (ephrin A) or a transmembrane domain (ephrin B). Ephrin receptors are likewise divided in two subclasses EphA and EphB, depending on the type of interaction with their ligands ephrin A or B. Ephrins and their receptors have been shown to play an essential role in vascular development during embryogenesis and in adult angiogenesis, as key regulators of vascular assembly, arteriovenous differentiation, and boundary formation. Both EphA and EphB receptors and their ligands are involved in vascular development. Especially, ephrin B2 is expressed in arterial endothelial cells, whereas its cognate receptor, EphB4 is expressed in venous endothelial cells. EphB4 (also named HTK) and its ligand, ephrin B2 (HTKL), play important roles in establishing and determining vascular networks. Blocking this receptor/ligand pair inhibits the end stage of tumour blood vessel formation, as well as causes direct growth inhibition of certain cancers.

EphB4 seems to be rather recalcitrant to inhibition. Despite its potential therapeutic importance, few non-peptidic small molecule inhibitors have been reported in the literature up to date (Miyazaki Y. et al., Bioorg Med Chem Lett 2007, 17:250-254; Zhao H. and Huang D., PLoS ONE, 2011, Volume 6, Issue 6; Martiny-Baron G. et al., Angiogenesis, 2010, Volume 13, Issue 3, 259-67).

The objective of the invention is to provide new 2-amino-1-phenyl-pyrrolo [3,2-b]quinoxaline-3-carboxamide derivates which particularly comprise a high selectivity to one or more protein kinases, and notably to one or more protein-tyrosine kinases, as well as pharmaceutical preparations thereof and their use in the treatment of cancer and intraocular neovascular syndromes.

This objective is attained by the subject-matter of the independent claims.

### SUMMARY OF THE INVENTION

An aspect of the invention relates to a compound characterized by a general formula (1),

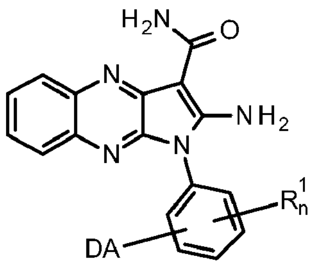

(1)

wherein n of $R^1_n$ is 0, 1, 2, 3 or 4, in particular n of $R^1_n$ is 0 or 1, and

- each $R^1$ independently from any other $R^1$ is $C_1$-$C_3$ alkyl, $C_1$-$C_3$ haloalkyl or $C_1$-$C_3$ alkoxy, in particular each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, and

DA is $-C(=O)NH_2$, -NHC(=O)OH, -NHC(=O)H, -C(=O)OH, $-NHC(=O)NH_2$, $-C(=O)NHR^2$, $-NHC(=O)R^2$, $-NHC(=O)NHR^2$, $-NHC(=O)NR^2_2$, $-C(=S)NH_2$, -NHC(=S)OH, -NHC(=S)H, -C(=S)OH, $-NHC(=S)NH_2$, $C_1$-$C_3$ thiol, $-C(=S)NHR^2$, $-NHC(=S)R^2$, $-NHC(=S)NHR^2$, $-NHC(=S)NR^2_2$ or $-NHC(=S)NR^2_2$, a substituted or unsubstituted $C_5$-$C_6$ heteroaryl or a substituted or unsubstituted $C_5$-$C_6$ saturated heterocycle, and

- $R^2$ is a substituted or unsubstituted $C_3$-$C_8$ cycloalkyl, a substituted or unsubstituted $C_3$-$C_8$ halocycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or
- $R^2$ is a substituent of the formula A

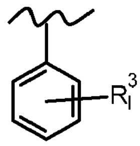

(formula A)

- with l of $R^3_l$ being 0, 1, 2, 3, 4 or 5, in particular l of $R^3_l$ being 0, 1 or 2, and
- each $R^3$ independently from any other $R^3$ $C_1$-$C_4$ alkyl, a $C_1$-$C_4$ alkoxy or a $C_1$-$C_4$ haloalkyl, a substituted or unsubstituted 5 or 6-membered heteroaryl ring, in particular $R^3$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl, F or a substituted or unsubstituted 5 or 6-membered heteroaryl ring.

Another aspect of the invention relates to a pharmaceutical preparation for treatment of cancer or intraocular neovascular syndromes, in particular for treatment of cancer, comprising at least one compound of the general formula (1).

A further aspect of the invention relates to a compound or a pharmaceutical preparation according to the invention for use in a method of treatment of disease.

Another aspect of the invention relates to a compound or a pharmaceutical preparation according to the invention for use in a method for treatment of cancer or intraocular neovascular syndromes, in particular for use in a method for treatment of cancer.

As used herein the term "parent molecule," refers to the 2-amino-1-phenyl-pyrrolo[3,2-b]quinoxaline-3-carboxamide basis structure

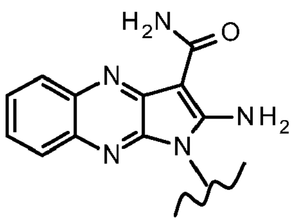

of the compounds of to the invention.

As used herein the term "substituted" refers to the addition of a substituent group to a parent compound.

"Substituent groups" can be protected or unprotected. Substituent groups may also be further substituted with other substituent groups and may be attached directly or by a linking group such as an alkyl, an amide or hydrocarbyl group to a parent compound. "Substituent groups" amenable herein include, without limitation, halogen, oxygen, nitrogen, sulphur, hydroxyl, alkyl, alkenyl, alkynyl, acyl ($-C(O)R_a$), carboxyl ($-C(O)OR_a$), aliphatic groups, alicyclic groups, alkoxy, substituted oxy ($-OR_a$), aryl, aralkyl, heterocyclic radical, heteroaryl, heteroarylalkyl, amino ($-N(R_b)(R_c)$), imino($=NR_b$), amido ($-C(O)N(R_b)(R_c)$ or $-N(Rb)C(O)R_a$), hydrazine derivates ($-C(NH)NR_aR_b$), tetrazole ($CN_4H_2$), azido ($-N_3$), nitro ($-NO_2$), cyano (-CN), isocyano (-NC), cyanato (-OCN), isocyanato (-NCO), thiocyanato (-SCN), isothio-cyanato (-NCS), carbamido ($-OC(O)N(R_b)(R_c)$ or $-N(R_b)C(O)OR_a$), thiol ($-SR_b$), sulfinyl ($-S(O)R_b$), sulfonyl ($-S(O)_2R_b$), sulfonamidyl ($-S(O)_2N(R_b)(R_c)$or $-N(R_b)S(O)_2R_b$) and fluorinated compounds $-CF_3$, $-OCF_3$, $-SCF_3$, $-SOCF_3$ or $-SO_2CF3$, wherein each Ra, Rb and Rc is, independently, H or a further substituent group with a preferred list including without limitation, H, alkyl, alkenyl, alkynyl, aliphatic, alkoxy, acyl, aryl, heteroaryl, alicyclic, heterocyclic and heteroarylalkyl.

As used herein the term "alkyl," refers to a saturated straight or branched hydrocarbon moiety containing 1 to 6, particularly 1 to 3 carbon atoms. Examples of alkyl groups include, without limitation, methyl, ethyl, (*n*-, *iso*-)propyl, (*n-, iso-, tert-*)butyl, n-hexyl, 2,3-methylbutyl and the like. Alkyl groups typically include from 1 to 3 carbon atoms ($C_1$-$C_3$ alkyl).

As used herein the term "cycloalkyl" refers to an interconnected alkyl group forming a ring structure containing 3 to 8, particularly 5 to 6 carbon atoms. Examples of cycloalkyl groups include, without limitation, cyclopropane, cyclopentane or cyclohexane and the like. Cycloalkyl groups typically include from 5 to 6 carbon atoms ($C_5$-$C_6$ cycloalkyl).

As used herein the term "haloalkyl," refers to a saturated straight or branched hydrocarbon moiety containing

1 to 6, particularly 1 to 3 carbon atoms and at least one halogen atom, in particular Cl or F, connected to a carbon atom. Examples of haloalkyl groups include, without limitation, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$ and the like. Haloalkyl groups typically include 1 to 3 carbon atoms ($C_1$-$C_3$ haloalkyl). The same applies for the term "halo cycloalkyl".

As used herein the term "saturated heterocycle" refers to cycloalkyl compounds in which at least one carbon atom is replaced with an oxygen, a nitrogen or a sulphur atom forming a non-aromatic structure. Saturated heterocycle groups as used herein may optionally charged and/or may include further substituent groups.

Alkyl or cycloalkyl groups as used herein may optionally include further substituent groups.

As used herein the term "alkoxy," refers to an oxygen-alkyl moiety, wherein the oxygen atom is used to attach the alkoxy group. Examples of alkoxy groups include without limitation, methoxy, ethoxy, propoxy, isopropoxy, n-butoxy, sec-butoxy, tert-butoxy, n-pentoxy, neopentoxy, n-hexoxy and the like. Alkoxy groups as used herein may optionally include further substituent groups.

As used herein the term "aryl" refers to a hydrocarbon with alternating double and single bonds between the carbon atoms forming a ring structure (in the following also "aromatic hydrocarbon"). The term "heteroaryl" refers to aryl compounds in which at least one carbon atom is replaced with an oxygen, a nitrogen or a sulphur atom. The aromatic hydrocarbon may be neutral or charged. Examples of aryl or hetero aryl groups are benzene, pyridine, pyrrole, quinoline or imidazole. Aryl or hetero aryl groups as used herein may optionally include further substituent groups.

As used herein the term "Donor-Acceptor" or "DA" refers to a moiety, which is able to provide a hydrogen bonding as a hydrogen donor and a hydrogen bonding as a hydrogen acceptor to an adjacent molecule.

**Detailed description of the invention**

According to one aspect the invention relates to compounds of the a general formula (1),

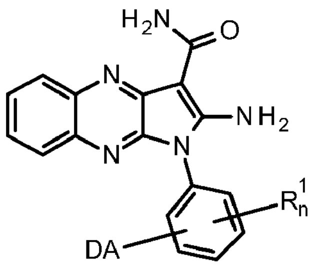

(1)

wherein n of $R^1_n$ is 0, 1, 2, 3 or 4, in particular n of $R^1_n$ is 0 or 1, and

- each $R^1$ independently from any other $R^1$ is $C_1$-$C_3$ alkyl, $C_1$-$C_3$ haloalkyl or $C_1$-$C_3$ alkoxy, in particular each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, and

DA is $-C(=O)NH_2$, -NHC(=O)OH, -NHC(=O)H, -C(=O)OH, $-NHC(=O)NH_2$, $-C(=O)NHR^2$, $-NHC(=O)R^2$, $-NHC(=O)NHR^2$, $-NHC(=O)NR^2_2$, $-C(=S)NH_2$, -NHC(=S)OH, -NHC(=S)H, -C(=S)OH, $-NHC(=S)NH_2$, $C_1$-$C_3$ thiol, $-C(=S)NHR^2$, $-NHC(=S)R^2$, $-NHC(=S)NHR^2$, $-NHC(=S)NR^2_2$ or $-NHC(=S)NR^2_2$, a substituted or unsubstituted $C_5$-$C_6$ heteroaryl or a substituted or unsubstituted $C_5$-$C_6$ saturated heterocycle, and

- $R^2$ is a substituted or unsubstituted $C_3$-$C_8$ cycloalkyl, a substituted or unsubstituted $C_3$-$C_8$ halocycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or

- $R^2$ is a substituent of the formula A

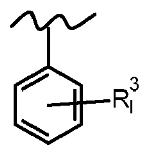

(formula A)

- with l of $R^3_l$ being 0, 1, 2, 3, 4 or 5, in particular l of $R^3_l$ being 0, 1 or 2, and

- each $R^3$ independently from any other $R^3$ $C_1$-$C_4$ alkyl, a $C_1$-$C_4$ alkoxy or a $C_1$-$C_4$ haloalkyl, a substituted or unsubstituted $C_5$-$C_6$ heteroaryl, in particular $R^3$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl, F or a substituted or unsubstituted $C_5$-$C_6$ heteroaryl, or a substituted or unsubstituted $C_5$-$C_6$ heteroaryl.

In some embodiments, DA is in meta position in relation to the attachment position of the benzene moiety to the parent moiety.
In some embodiments, DA is in para position in relation to the attachment position of the benzene moiety to the parent moiety.
In some embodiments, n is 0.
In some embodiments, n is 0 and DA is in meta position in relation to the attachment position of the benzene moiety to the parent moiety.
In some embodiments, n is 0 and DA is in para position in relation to the attachment position of the benzene moiety to the parent moiety.
In some embodiments, n is 1.
In some embodiments, n is 1 and $R^1$ is in ortho position in relation to the attachment position of the benzene moiety.
In some embodiments, n is 1 and $R^1$ is in ortho position in relation to the attachment position of the benzene moiety and DA is in meta position in relation to the attachment position of the benzene moiety to the parent moiety.
In some embodiments, n is 1 and $R^1$ is in ortho position in relation to the attachment position of the benzene moiety and DA is in para position in relation to the attachment position of the benzene moiety to the parent moiety.
In some embodiments, n is 1 and $R^1$ is in ortho and DA is in meta position in relation to the attachment position of the benzene moiety to the parent moiety yielding a 1, 2, 5 substitution pattern.
In some embodiments, $R^1$ is $CH_3$ or F.
In some embodiments, n is 1 and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, and in particular $R^1$ is $CH_3$, Cl or F, and more particularly $R^1$ is $CH_3$ or F.
In some embodiments, DA is $-C(=O)NH_2$, $-NHC(=O)OH$, $-NHC(=O)H$, $-C(=O)OH$, $-NHC(=O)NH_2$, $-NHC(=S)NH_2$, a substituted or unsubstituted diazole or a substituted or unsubstituted triazole, in particular a triazole with one carbon atom being connected to the benzene moiety and the other one comprising H or $CH_3$, in particular DA is $-C(=O)NH_2$, $-NHC(=O)OH$, $-NHC(=O)NH_2$ or 3-methyl-1,2,4-triazole.
In some embodiments, DA is $-C(=O)NH_2$, $-NHC(=O)OH$, $-NHC(=O)H$, $-C(=O)OH$, $-NHC(=O)NH_2$, $-NHC(=S)NH_2$, in particular DA is $-C(=O)NH_2$, $-NHC(=O)OH$, $-NHC(=O)NH_2$.
In some embodiments, DA is $-C(=O)NHR^2$, $-NHC(=O)R^2$, $-NHC(=O)NHR^2$, $-NHC(=S)NHR^2$ or $-NHC(=O)NR^2_2$, in particular DA is $-C(=O)NHR^2$, $-NHC(=O)R^2$, $-NHC(=O)NHR^2$ or - $NHC(=S)NHR^2$.
In some embodiments, DA is $-C(=O)NHR^2$, $-NHC(=O)R^2$, or $C(=O)NR^2_2$ and DA is in meta position in relation to the attachment position of the benzene moiety.
In some embodiments, DA is $-NHC(=O)NHR^2$ or$-NHC(=S)NHR^2$ and DA is in para position in relation to the attachment position of the benzene moiety.
In some embodiments, DA is a substituted or unsubstituted diazole or a substituted or unsubstituted triazole, in particular a triazole with one carbon atom being connected to the benzene moiety and the other one comprising H or $CH_3$, and more particularly DA is 3-methyl-1,2,4-triazole.
In some embodiments, $R^2$ is a substituted or unsubstituted $C_3$-$C_8$ cycloalkyl, a substituted or unsubstituted $C_3$-$C_8$ halo cycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl.
In some embodiments, $R^2$ is a substituted or unsubstituted $C_5$-$C_8$ cycloalkyl, a substituted or unsubstituted $C_5$-$C_8$ halo cycloalkyl, a substituted or unsubstituted $C_5$-$C_8$ saturated heterocycle, in particular a substituted or unsubstituted $C_5$-$C_6$ saturated heterocycle, a substituted or unsubstituted $C_3$-$C_8$ heteroaryl, in particular a substituted or unsubstituted $C_5$-$C_6$ heteroaryl
In some embodiments, $R^2$ is a substituted or unsubstituted $C_5$-$C_6$ saturated heterocycle or a substituted or unsubstituted $C_5$-$C_6$ heteroaryl, in particular a substituted or unsubstituted imidazole or a substituted or unsubstituted pyrazole.
In some embodiments, $R^2$ is a substituent of the formula A with l of $R^3_l$ being 0, 1, 2, 3, 4 or 5, in particular l of $R^3$, being 0, 1 or 2, and each $R^3$ independently from any other $R^3$ is a $C_1$-$C_4$ alkyl, a $C_1$-$C_4$ alkoxy or a $C_1$-$C_4$ haloalkyl, a substituted or unsubstituted $C_5$-$C_6$ heteroaryl, in particular $R^3$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl, F or a substituted or unsubstituted $C_5$-$C_6$

heteroaryl.

In some embodiments, $R^3$ is $CH_3$, $CH_2F$, $CHF_2$, $CF_3$, $CH_2CF_3$, $CH_2CHF_2$, $CH_2CH_2F$, $OCH_3$, Cl or F.

In some embodiments, $R^3$ is $CF_3$ or a substituted or unsubstituted imidazole connected to the benzene moiety of $R^2$ in position 1, in particular a substituted imidazole comprising a $C_1$-$C_6$ alkyl, in particular a $CH_3$, in position 4.

In some embodiments, each $R^3$ is in meta position in relation to the attachment position of the benzene moiety of $R^2$ to DA,

In some embodiments, $R^2$ is a substituted or unsubstituted $C_5$-$C_6$ heteroaryl, in particular a substituted or unsubstituted $C_5$ heteroaryl, more particularly substituted or unsubstituted imidazole or a substituted or unsubstituted pyrazole.

In some embodiments, $R^2$ is a substituted or unsubstituted pyrazole, in particular a pyrazole connected to the benzene moiety of $R^2$ in position 5.

In some embodiments, $R^2$ is a substituted pyrazole comprising a $C_1$-$C_6$ alkyl, in particular t-butyl in position 3, and/or a substituted or unsubstituted $C_6$ to $C_{14}$ aryl or heteroaryl group, wherein in particular said $C_6$ to $C_{14}$ aryl or heteroaryl group is connected to said pyrazole in position 1.

In some embodiments, $R^2$ is a substituted pyrazole comprising a $C_1$-$C_6$ alkyl, in particular t-butyl in position 3, and/or a substituted or unsubstituted $C_6$ to $C_{14}$ aryl or heteroaryl group connected to said pyrazole in position 1, wherein in particular said $C_6$ to $C_{14}$ aryl or heteroaryl group is a substituted or unsubstituted quinoline, in particular an unsubstituted quinoline.

In some embodiments, $R^2$ is a substituted pyrazole comprising a $C_1$-$C_6$ alkyl, in particular t-butyl in position 3, and/or a substituted or unsubstituted $C_6$ to $C_{14}$ aryl or heteroaryl group connected to said pyrazole in position 1, wherein in particular said $C_6$ to $C_{14}$ aryl or heteroaryl group is a substituted or unsubstituted phenyl group, in particular a substituted phenyl group comprising a $C_1$-$C_6$ alkyl, in particular $C_1$-alkyl, wherein in particular said $C_1$-$C_6$ alkyl is situated in meta position in relation to the attachment position of the phenyl group to said pyrazole.

In some embodiments, n of $R^1_n$ is 0, 1, 2, 3 or 4, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$ $CHFCF_3$, $CHFCHF_2$, $CHFCH_2F$, $CF_2CF_3$, $CF_2CHF_2$, $CF_2CH_2F$, Cl or F, and DA is

- -C(=O)$NH_2$, -NHC(=O)OH, -NHC(=O)H, -C(=O)OH, -NHC(=O)$NH_2$, -NHC(=S)$NH_2$ or a substituted or unsubstituted $C_5$-$C_6$ heteroaryl or a substituted or unsubstituted $C_5$-$C_6$ saturated heterocycle, in particular a triazole with one carbon atom being connected to the benzene moiety and the other one comprising H or $CH_3$, or

- -C(=O)$NH_2$, -NHC(=O)OH, -NHC(=O)$NH_2$ or 3-methyl-1 ,2,4-triazole.

wherein in particular DA is in meta or para, in particular in meta, position in relation to the attachment position of the benzene moiety to the parent moiety.

In some embodiments, n of $R^1_n$ is 0 or 1, and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$ Cl or F, in particular $CH_3$, Cl or F and DA is

- -C(=O)$NH_2$, -NHC(=O)OH, -NHC(=O)H, -C(=O)OH, -NHC(=O)$NH_2$, -NHC(=S)$NH_2$ or a triazole with one carbon atom being connected to the benzene moiety and the other one comprising H or $CH_3$, or

- -C(=O)$NH_2$, -NHC(=O)OH, -NHC(=O)$NH_2$ or 3-methyl-1 ,2,4-triazole,

wherein in particular DA is in meta or para, in particular in meta, position in relation to the attachment position of the benzene moiety to the parent moiety.

In some embodiments, n of $R^1_n$ is 1, and $R^1$ is $CH_3$, $OCH_3$, $CF_3$, $CHF_2$, $CH_2F$, $CH_2CF_3$, $CH_2CHF_2$, $CH_2CH_2F$ $CHFCF_3$, $CHFCHF_2$, $CHFCH_2F$, $CF_2CF_3$, $CF_2CHF_2$, $CF_2CH_2F$, Cl or F, in particular $CH_3$, Cl or F, and DA is

- -C(=O)$NH_2$, -NHC(=O)OH, -NHC(=O)H, -C(=O)OH, -NHC(=O)$NH_2$, -NHC(=S)$NH_2$, or a triazole with one carbon atom being connected to the benzene moiety and the other one comprising H or $CH_3$, or

- -C(=O)$NH_2$, -NHC(=O)OH, -NHC(=O)$NH_2$ or 3-methyl-1 ,2,4-triazole, and

$R^1$ is in ortho position in relation to the attachment position of the benzene moiety and/or DA is in meta or para, in particular in meta, position in relation to the attachment position of the benzene moiety to the parent moiety.

In some embodiments, n of $R^1_n$ is 1, and $R^1$ is $CH_3$, $OCH_3$, $CF_3$, $CHF_2$, $CH_2F$, $CH_2CF_3$, $CH_2CHF_2$, $CH_2CH_2F$ $CHFCF_3$, $CHFCHF_2$, $CHFCH_2F$, $CF_2CF_3$, $CF_2CHF_2$, $CF_2CH_2F$, Cl or F, in particular $CH_3$, Cl or F , and DA is

- $-C(=O)NH_2$, $-NHC(=O)OH$, $-NHC(=O)H$, $-C(=O)OH$, $-NHC(=O)NH_2$, $-NHC(=S)NH_2$ or a triazole with one carbon atom being connected to the benzene moiety and the other one comprising H or $CH_3$, or

- $-C(=O)NH_2$, $-NHC(=O)OH$, $-NHC(=O)NH_2$ or 3-methyl-1 ,2,4-triazole, and

$R^1$ is in ortho and DA is in meta position in relation to the attachment position of the benzene moiety yielding a 1, 2, 5 substitution pattern.

In some embodiments, n of $R^1_n$ is 1, and $R^1$ is $CH_3$, Cl or F, and DA is a triazole, in particular 3-methyl-1,2,4-triazole, wherein in particular $R^1$ is in ortho position and/or DA is in meta position in relation to the attachment position of the benzene moiety to the parent moiety, wherein more particularly $R^1$ is in ortho and DA is in meta position yielding a 1, 2, 5 substitution pattern.

In some embodiments, n of $R^1_n$ is 0, and DA is a triazole, in particular 3-methyl-1,2,4-triazole, wherein in particular DA is in meta or para, in particular in meta, position in relation to the attachment position of the benzene moiety to the parent moiety.

In some embodiments, DA is $-C(=O)NHR^2$, $-NHC(=O)R^2$, $-NHC(=O)NHR^2$ $-NHC(=S)NHR^2$ or$-NHC(=O)NR2^2$, in particular DA is $-C(=O)NHR^2$, $-NHC(=O)R^2$, $-NHC(=O)NHR^2$ or $-NHC(=S)NHR^2$, more particularly DA is $-C(=O)NHR^2$, $-NHC(=O)R^2$,$-NHC(=O)NHR^2$, $-NHC(=S)NHR^2$, and $R^2$ is

- a substituted or unsubstituted $C_3$-$C_8$ cycloalkyl, a substituted or unsubstituted $C_3$-$C_8$ halo cycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_8$ cycloalkyl, a substituted or unsubstituted $C_5$-$C_8$ halo cycloalkyl, a substituted or unsubstituted $C_5$-$C_8$ saturated heterocycle, a substituted or unsubstituted $C_5$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_6$ saturated heterocycle or a substituted or unsubstituted $C_5$-$C_6$ heteroaryl, in particular a substituted or unsubstituted imidazole or a substituted or unsubstituted pyrazole,

wherein in particular DA is in meta or para, in particular in meta, position in relation to the attachment position of the benzene moiety.

In some embodiments, n of $R^1_n$ is 0, 1, 2, 3 or 4, in particular n of $R^1_n$ is 0 or 1, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, and DA is $-C(=O)NHR^2$, $-NHC(=O)R^2$, $-NHC(=O)NHR^2$ $-NHC(=S)NHR^2$, $C(=O)NR^2_2$, or $-NHC(=O)NR^2_2$, in particular DA is $-C(=O)NHR^2$, $-NHC(=O)R^2$, $-NHC(=O)NHR^2$ or$-NHC(=S)NHR^2$, and $R^2$ is

- a substituted or unsubstituted $C_3$-$C_8$ cycloalkyl, a substituted or unsubstituted $C_3$-$C_8$ halo cycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_8$ cycloalkyl, a substituted or unsubstituted $C_5$-$C_8$ halo cycloalkyl, a substituted or unsubstituted $C_5$-$C_8$ saturated heterocycle, a substituted or unsubstituted $C_5$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_6$ saturated heterocycle or a substituted or unsubstituted $C_5$-$C_6$ heteroaryl, in particular a substituted or unsubstituted imidazole or a substituted or unsubstituted pyrazole,

wherein in particular DA is in meta or para, in particular in meta, position and/or $R^1$ is in ortho position in relation to the attachment position of the benzene moiety to the parent moiety.

In some embodiments, n of $R^1_n$ is 1, and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, in particular $CH_3$, Cl or F, and DA is $-C(=O)NHR^2$, $-NHC(=O)R^2$, $-NHC(=O)NHR^2$$-NHC(=S)NHR^2$, $C(=O)NR^2_2$ or$-NHC(=O)NR2^2$, in particular DA is $-C(=O)NHR^2$,$-NHC(=O)R^2$, $-NHC(=O)NHR^2$ or $-NHC(=S)NHR^2$, and $R^2$ is

- a substituted or unsubstituted $C_3$-$C_8$ cycloalkyl, a substituted or unsubstituted $C_3$-$C_8$ halo cycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_8$ cycloalkyl, a substituted or unsubstituted $C_5$-$C_8$ halo cycloalkyl, a substituted or unsubstituted $C_5$-$C_8$ saturated heterocycle, a substituted or unsubstituted $C_5$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_6$ saturated heterocycle or a substituted or unsubstituted $C_5$-$C_6$ heteroaryl, in particular a substituted or unsubstituted imidazole or a substituted or unsubstituted pyrazole, and

wherein in particular $R^1$ is in ortho position and/or DA is in meta or para, in particular in meta, position in relation to the attachment position of the benzene moiety to the parent moiety, wherein more particularly $R^1$ is in ortho and DA is in meta position in relation to the attachment position of the benzene moiety yielding a 1, 2, 5 substitution pattern.

In some embodiments, n of $R^1_n$ is 0, and DA is -C(=O)NHR$^2$, -NHC(=O)R$^2$, -NHC(=O)NHR$^2$ -NHC(=S)NHR$^2$, C(=O)NR$^2_2$ or -NHC(=O)NR$^2_2$, in particular and $R^2$ is

- a substituted or unsubstituted $C_3$-$C_8$ cycloalkyl, a substituted or unsubstituted $C_3$-$C_8$ halo cycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_8$ cycloalkyl, a substituted or unsubstituted $C_5$-$C_8$ halo cycloalkyl, a substituted or unsubstituted $C_5$-$C_8$ saturated heterocycle, a substituted or unsubstituted $C_5$-$C_8$ heteroaryl, a $C_1$-$C_6$ alkyl, a $C_1$-$C_6$ alkoxy or a $C_1$-$C_6$ haloalkyl, or

- a substituted or unsubstituted $C_5$-$C_6$ saturated heterocycle or a substituted or unsubstituted $C_5$-$C_6$ heteroaryl, in particular a substituted or unsubstituted imidazole or a substituted or unsubstituted pyrazole, and

wherein in particular DA is in meta or para, in particular in meta, position in relation to the attachment position of the benzene moiety to the parent moiety.

In some embodiments, I is 0.

In some embodiments, I is 1 and $R^3$ is $CF_3$, F or 4-methyl-imidazole, in particular $CF_3$ or 4-methyl-imidazole.

In some embodiments, n of $R^1_n$ is 0, 1, 2, 3 or 4, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, DA is -C(=O)NHR$^2$,-NHC(=O)R$^2$, -NHC(=O)NHR$^2$, -NHC(=S)NHR$^2$, C(=O)NR$^2_2$ or-NHC(=O)NR2$^2$, in particular DA is -C(=O)NHR$^2$, -NHC(=O)R$^2$, -NHC(=O)NHR$^2$ or -NHC(=S)NHR$^2$, and

$R^2$ is a substituent of the formula A

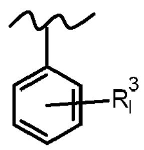

(formula A)

with I of $R^3_l$ being 0, 1, 2, 3, 4 or 5, in particular I of $R^3_l$ being 0, 1 or 2, and each $R^3$ independently from any other $R^3$ is

- a $C_1$-$C_4$ alkyl, a $C_1$-$C_4$ alkoxy or a $C_1$-$C_4$ haloalkyl, a substituted or unsubstituted $C_5$-$C_6$ heteroaryl, or

- a substituted or unsubstituted $C_5$-$C_6$ heteroaryl, in particular a substituted or unsubstituted imidazole, $CH_3$, $CH_2F$, $CHF_2$, $CF_3$, $CH_2CF_3$, $CH_2CHF_2$, $CH_2CH_2F$, $OCH_3$, Cl or F, or

- $CF_3$, or

- a substituted or unsubstituted $C_5$-$C_6$ heteroaryl, in particular a substituted or unsubstituted imidazole, or

- a substituted or unsubstituted imidazole connected to the benzene moiety of $R^2$ in position 1, in particular a substituted imidazole comprising a $C_1$-$C_6$ alkyl, in particular a $C_1$-alkyl, in position 4,

wherein in particular each $R^3$ is in meta position in relation to the attachment position of the benzene moiety of $R^2$ to DA and/or $R^1$ is in ortho position in relation to the attachment position of the benzene moiety, wherein more particularly DA is in meta or para, in particular in meta, position in relation to the attachment position of the benzene moiety to the

parent moiety.

In some embodiments, n of $R^1_n$ is 0 or 1, and $R^1$ is $CH_3$, $OCH_3$, $CF_3$, $CHF_2$, $CH_2F$, $CH_2CF_3$, $CH_2CHF_2$, $CH_2CH_2F$, Cl or F, in particular $CH_3$, Cl or F, and DA is -C(=O)NHR$^2$, -NHC(=O)R$^2$, -NHC(=O)NHR$^2$-NHC(=S)NHR$^2$, C(=O)NR$^2_2$ or -NHC(=O)NR2$^2$, in particular DA is -C(=O)NHR$^2$, -NHC(=O)R$^2$, -NHC(=O)NHR$^2$ or -NHC(=S)NHR$^2$, and $R^2$ is of the formula A with l of $R^3_l$ being 0, 1, 2, 3, 4 or 5, in particular l of $R^3_l$ being 0, 1 or 2, and each $R^3$ independently from any other $R^3$ is

- a $C_1$-$C_4$ alkyl, a $C_1$-$C_4$ alkoxy or a $C_1$-$C_4$ haloalkyl, a substituted or unsubstituted $C_5$-$C_6$ heteroaryl, or
- a substituted or unsubstituted $C_5$-$C_6$ heteroaryl, in particular a substituted or unsubstituted imidazole, $CH_3$, $CH_2F$, $CHF_2$, $CF_3$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, $OCH_3$, Cl or F, or
- $CF_3$, or
- a substituted or unsubstituted $C_5$-$C_6$ heteroaryl, in particular a substituted or unsubstituted imidazole, or
- a substituted or unsubstituted imidazole connected to the benzene moiety of $R^2$ in position 1, in particular a substituted imidazole comprising a $C_1$-$C_6$ alkyl, in particular a $C_1$-alkyl, in position 4, and

wherein in particular each $R^3$ is in meta position in relation to the attachment position of the benzene moiety of $R^2$ to DA and/or $R^1$ is in ortho position in relation to the attachment position of the benzene moiety, wherein more particularly DA is in meta or para, in particular in meta, position in relation to the attachment position of the benzene moiety to the parent moiety.

In some embodiments, n of $R^1_n$ is 0 or 1, and $R^1$ is $CH_3$, Cl or F, and DA is -C(=O)NHR$^2$, -NHC(=O)R$^2$, -NHC(=O)NHR$^2$ or -NHC(=S)NHR$^2$, and $R^2$ is of the formula A with l of $R^3_l$ being 0, 1, 2, 3, 4 or 5, in particular l of $R^3_l$ being 0, 1 or 2, and each $R^3$ independently from any other $R^3$ is

- a $C_1$-$C_4$ alkyl, a $C_1$-$C_4$ alkoxy or a $C_1$-$C_4$ haloalkyl, a substituted or unsubstituted $C_5$-$C_6$ heteroaryl, or
- a substituted or unsubstituted $C_5$-$C_6$ heteroaryl, in particular a substituted or unsubstituted imidazole, $CH_3$, $CH_2F$, $CHF_2$, $CF_3$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, $OCH_3$, Cl or F, or
- $CF_3$, or
- a substituted or unsubstituted $C_5$-$C_6$ heteroaryl, in particular a substituted or unsubstituted imidazole, or
- a substituted or unsubstituted imidazole connected to the benzene moiety of $R^2$ in position 1, in particular a substituted imidazole comprising a $C_1$-$C_6$ alkyl, in particular a $C_1$-alkyl, in position 4,

wherein in particular each $R^3$ is in meta position in relation to the attachment position of the benzene moiety of $R^2$ to DA and/or $R^1$ is in ortho position in relation to the attachment position of the benzene moiety, wherein more particularly DA is in meta or para, in particular in meta, position in relation to the attachment position of the benzene moiety to the parent moiety.

In some embodiments, n of $R^1_n$ is 0 or 1, and $R^1$ is $CH_3$, Cl or F, and DA is -C(=O)NHR$^2$,-NHC(=O)R$^2$, -NHC(=O)NHR$^2$ or -NHC(=S)NHR$^2$, and $R^2$ is of the formula A with

- l of $R^3_l$ being 1, and $R^3$ being
  - a $C_1$-$C_4$ alkyl, a $C_1$-$C_4$ alkoxy or a $C_1$-$C_4$ haloalkyl, a substituted or unsubstituted $C_5$-$C_6$ heteroaryl, or
  - a substituted or unsubstituted $C_5$-$C_6$ heteroaryl, in particular a substituted or unsubstituted imidazole, $CH_3$, $CH_2F$, $CHF_2$, $CF_3$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, $OCH_3$, Cl or F, or
  - $CF_3$, or

- a substituted or unsubstituted $C_5$-$C_6$ heteroaryl, in particular a substituted or unsubstituted imidazole, or

- a substituted or unsubstituted imidazole connected to the benzene moiety of $R^2$ in position 1, in particular a substituted imidazole comprising a $C_1$-$C_6$ alkyl, in particular a $C_1$-alkyl, in position 4, or

- I of $R^3$, being 2, and each $R^3$ independently from any other $R^3$ being

  - a $C_1$-$C_4$ alkyl, a $C_1$-$C_4$ alkoxy or a $C_1$-$C_4$ haloalkyl, a substituted or unsubstituted $C_5$-$C_6$ heteroaryl, or

  - a substituted or unsubstituted $C_5$-$C_6$ heteroaryl, in particular a substituted or unsubstituted imidazole, $CH_3$, $CH_2F$, $CHF_2$, $CF_3$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$,, $OCH_3$, Cl or F, or

  - a substituted or unsubstituted $C_5$-$C_6$ heteroaryl, in particular a substituted or unsubstituted imidazole, or

  - a substituted or unsubstituted imidazole connected to the benzene moiety of $R^2$ in position 1, in particular a substituted imidazole comprising a $C_1$-$C_6$ alkyl, in particular a $C_1$-alkyl, in position 4, or

  - each $R^3$ being $CF_3$,

wherein in particular each $R^3$ is in meta position in relation to the attachment position of the benzene moiety of $R^2$ to DA and/or $R^1$ is in ortho position in relation to the attachment position of the benzene moiety, wherein more particularly DA is in meta or para, in particular in meta, position in relation to the attachment position of the benzene moiety to the parent moiety.

In some embodiments, n of $R^1_n$ is 1, and $R^1$ is $CH_3$, Cl or F, and DA is C(=O)NH$R^2$, -NHC(=O)$R^2$ or -NHC(=O)NH$R^2$, and $R^2$ is of the formula A with

- I of $R^3_l$ being 1, and $R^3$ being

  - a substituted imidazole connected to the benzene moiety of $R^2$ in position 1 comprising a $C_1$-$C_6$ in position 4, in particular said imidazole is 4-methyl imidazole, or $CF_3$, in particular with $R^3$ being $CF_3$, or

- I of $R^3_l$ being 2, and each $R^3$ independently from any other $R^3$ being

  - a substituted imidazole connected to the benzene moiety of $R^2$ in position 1 comprising a $C_1$-$C_6$ in position 4, in particular said imidazole is 4-methyl imidazole, or $CF_3$, in particular with $R^3$ being $CF_3$, or

- I of $R^3_l$ being 2, and one of $R^3$ being a substituted imidazole connected to the benzene moiety of $R^2$ in position 1 comprising a $C_1$-$C_6$ in position 4, in particular said imidazole is 4-methyl imidazole, and the other one of $R^3$ being $CF_3$, and

wherein DA is in meta or para, in particular in meta, position in relation to the attachment position of the benzene moiety to the parent moiety, and each $R^3$ is in meta position in relation to the attachment position of the benzene moiety of $R^2$ to DA and $R^1$ is in ortho position in relation to the attachment position of the benzene moiety.

In some embodiments, n of $R^1_n$ is 0, and DA is C(=O)NH$R^2$, -NHC(=O)$R^2$ or -NHC(=O)NH$R^2$, and $R^2$ is of the formula A with

- I of $R^3_l$ being 1, and $R^3$ being

  - a substituted imidazole connected to the benzene moiety of $R^2$ in position 1 comprising a $C_1$-$C_6$ in position 4, in particular said imidazole is 4-methyl imidazole, or $CF_3$, in particular with $R^3$ being $CF_3$, or

- I of $R^3_l$ being 2, and each $R^3$ independently from any other $R^3$ being

  - a substituted imidazole connected to the benzene moiety of $R^2$ in position 1 comprising a $C_1$-$C_6$ in position 4, in particular said imidazole is 4-methyl imidazole, or $CF_3$, in particular with $R^3$ being $CF_3$, or

- l of $R^3_l$ being 2, and one of $R^3$ being a substituted imidazole connected to the benzene moiety of $R^2$ in position 1 comprising a $C_1$-$C_6$ in position 4, in particular said imidazole is 4-methyl imidazole, and the other one of $R^3$ being $CF_3$, and

  wherein DA is in meta or para, in particular in meta, position in relation to the attachment position of the benzene moiety to the parent moiety, and each $R^3$ is in meta position in relation to the attachment position of the benzene moiety of $R^2$ to DA.

In some embodiments, n of $R^1_n$ is 0, 1, 2, 3 or 4, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, DA is -C(=O)NH$R^2$, -NHC(=O)$R^2$, -NHC(=O)NH$R^2$-NHC(=S)NH$R^2$, C(=O)N$R^2_2$ or-NHC(=O)N$R_2^2$, in particular DA is -C(=O)NH$R^2$, -NHC(=O)$R^2$, -NHC(=O)NH$R^2$ or -NHC(=S)NH$R^2$, and $R^2$ is a substituted or unsubstituted $C_5$-$C_6$ heteroaryl, in particular a substituted or unsubstituted $C_5$ heteroaryl, and said substituted $C_5$-$C_6$ heteroaryl or substituted $C_5$ heteroaryl comprises

- a $C_1$-$C_6$ alkyl, in particular t-butyl, substitution, and/or
- a substituted or unsubstituted $C_6$ to $C_{14}$ aryl or heteroaryl group, in particular a substituted or unsubstituted quinoline, more particularly a unsubstituted quinoline, and

wherein in particular $R^1$ is in ortho position in relation to the attachment position of the benzene moiety, wherein more particularly DA is in meta or para, in particular in para, position in relation to the attachment position of the benzene moiety to the parent moiety.

In some embodiments, n of $R^1_n$ is 0 or 1, and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, in particular $CH_3$, Cl or F, and DA is -C(=O)NH$R^2$, -NHC(=O)$R^2$, -NHC(=O)NH$R^2$, -NHC(=S)NH$R^2$ or-NHC(=O)N$R_2^2$, in particular DA is -C(=O)NH$R^2$, -NHC(=O)$R^2$, - NHC(=O)NH$R^2$ or -NHC(=S)NH$R^2$, and $R^2$ is a substituted or unsubstituted $C_5$-$C_6$ heteroaryl, in particular a substituted or unsubstituted $C_5$ heteroaryl, and said substituted $C_5$-$C_6$ heteroaryl or substituted $C_5$ heteroaryl comprises

- a $C_1$-$C_6$ alkyl, in particular t-butyl, substitution, and/or
- a substituted or unsubstituted $C_6$ to $C_{14}$ aryl or heteroaryl group, in particular a substituted or unsubstituted quinoline, more particularly a unsubstituted quinoline, and

wherein in particular $R^1$ is in ortho position in relation to the attachment position of the benzene moiety, wherein more particularly DA is in meta or para, in particular in para, position in relation to the attachment position of the benzene moiety to the parent moiety.

In some embodiments, n of $R^1_n$ is 0 or 1, and $R^1$ is $CH_3$, Cl or F, and DA is -C(=O)NH$R^2$, -NHC(=O)$R^2$, -NHC(=O)NH$R^2$ or -NHC(=S)NH$R^2$, and $R^2$ is a substituted or unsubstituted $C_5$-$C_6$ heteroaryl, in particular a substituted or unsubstituted $C_5$ heteroaryl, and said substituted $C_5$-$C_6$ heteroaryl or substituted $C_5$ heteroaryl comprises

- a $C_1$-$C_6$ alkyl, in particular t-butyl, substitution, and/or
- a substituted or unsubstituted $C_6$ to $C_{14}$ aryl or heteroaryl group, in particular a substituted or unsubstituted quinoline, more particularly a unsubstituted quinoline, and

wherein in particular $R^1$ is in ortho position in relation to the attachment position of the benzene moiety, wherein more particularly DA is in meta or para, in particular in para, position in relation to the attachment position of the benzene moiety to the parent moiety.

In some embodiments, n of $R^1_n$ is 0 or 1, and $R^1$ is $CH_3$, Cl or F, and DA is -C(=O)NH$R^2$, -NHC(=O)$R^2$, -NHC(=O)NH$R^2$ or -NHC(=S)NH$R^2$, and $R^2$ is a substituted or unsubstituted $C_5$-$C_6$ heteroaryl, in particular a substituted or unsubstituted $C_5$ heteroaryl, and said substituted $C_5$-$C_6$ heteroaryl or substituted $C_5$ heteroaryl comprises

- a $C_1$-$C_6$ alkyl, in particular t-butyl, substitution, and/or
- a substituted or unsubstituted $C_6$ to $C_{14}$ aryl or heteroaryl group, in particular a substituted or unsubstituted quinoline, more particularly a unsubstituted quinoline, and

wherein in particular $R^1$ is in ortho position in relation to the attachment position of the benzene moiety, wherein more particularly DA is in meta or para, in particular in para, position in relation to the attachment position of the benzene

moiety to the parent moiety. In some embodiments, n of $R^1_n$ is 0, 1, 2, 3 or 4, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, DA is -C(=O)NHR$^2$, -NHC(=O)R$^2$, -NHC(=O)NHR$^2$ -NHC(=S)NHR$^2$ or -NHC(=O)NR$^2_2$, in particular DA is - C(=O)NHR$^2$, -NHC(=O)R$^2$, -NHC(=O)NHR$^2$ or-NHC(=S)NHR$^2$, and $R^2$ is a substituted or unsubstituted pyrazole, in particular a pyrazole connected to the benzene moiety of $R^2$ in position 5, and said pyrazole comprises

- a $C_1$-$C_6$ alkyl substitution in position 3, in particular t-butyl substitution in position 3, and/or

- a substituted or unsubstituted $C_6$ to $C_{14}$ aryl or heteroaryl group in position 1, in particular a substituted or unsubstituted quinoline in position 1, more particularly a unsubstituted quinoline in position 1, and

wherein in particular $R^1$ is in ortho position in relation to the attachment position of the benzene moiety, wherein more particularly DA is in meta or para, in particular in para, position in relation to the attachment position of the benzene moiety to the parent moiety.

In some embodiments, n of $R^1_n$ is 0 or 1, and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, in particular $CH_3$, Cl or F, and DA is -C(=O)NHR$^2$, -NHC(=O)R$^2$, -NHC(=O)NHR$^2$, -NHC(=S)NHR$^2$ or -NHC(=O)NR$^2_2$ in particular DA is -C(=O)NHR$^2$, -NHC(=O)R$^2$, - NHC(=O)NHR$^2$ or-NHC(=S)NHR$^2$, and $R^2$ is a substituted or unsubstituted pyrazole, in particular a pyrazole connected to the benzene moiety of $R^2$ in position 5, and said pyrazole comprises

- a $C_1$-$C_6$ alkyl substitution in position 3, in particular t-butyl substitution in position 3, and/or

- a substituted or unsubstituted $C_6$ to $C_{14}$ aryl or heteroaryl group in position 1, in particular a substituted or unsubstituted quinoline in position 1, more particularly a unsubstituted quinoline in position 1, and

wherein in particular $R^1$ is in ortho position in relation to the attachment position of the benzene moiety, wherein more particularly DA is in meta or para, in particular in para, position in relation to the attachment position of the benzene moiety to the parent moiety. In some embodiments, n of $R^1_n$ is 0 or 1, and $R^1$ is $CH_3$, Cl or F, and DA is -C(=O)NHR$^2$, -NHC(=O)R$^2$, -NHC(=O)NHR$^2$ or-NHC(=S)NHR$^2$, and $R^2$ is a substituted or unsubstituted pyrazole, in particular a pyrazole connected to the benzene moiety of $R^2$ in position 5, and said pyrazole comprises

- a $C_1$-$C_6$ alkyl substitution in position 3, in particular a t-butyl substitution in position 3, and/or
- a substituted or unsubstituted $C_6$ to $C_{14}$ aryl or heteroaryl group in position 1, in particular a substituted or unsubstituted quinoline in position 1, more particularly a unsubstituted quinoline in position 1, and

wherein in particular $R^1$ is in ortho position in relation to the attachment position of the benzene moiety, wherein more particularly DA is in meta or para, in particular in para, position in relation to the attachment position of the benzene moiety to the parent moiety.

In some embodiments, n of $R^1_n$ is 0, and DA is C(=O)NHR$^2$, -NHC(=O)R$^2$ or -NHC(=O)NHR$^2$, and $R^2$ is a substituted or unsubstituted pyrazole, in particular a pyrazole connected to the benzene moiety of $R^2$ in position 5, and said pyrazole comprises

- a $C_1$-$C_6$ alkyl substitution in position 3, in particular a t-butyl substitution in position 3, and/or

- a substituted or unsubstituted $C_6$ to $C_{14}$ aryl or heteroaryl group in position 1, in particular a substituted or unsubstituted quinoline in position 1, more particularly a unsubstituted quinoline in position 1, and

wherein in particular DA is in meta or para, in particular in para, position in relation to the attachment position of the benzene moiety to the parent moiety.

In some embodiments, n of $R^1_n$ is 1, and $R^1$ is $CH_3$, Cl or F, and DA is C(=O)NHR$^2$, -NHC(=O)R$^2$ or -NHC(=O)NHR$^2$, and $R^2$ is a substituted or unsubstituted pyrazole, in particular a pyrazole connected to the benzene moiety of $R^2$ in position 5, and said pyrazole comprises

- a $C_1$-$C_6$ alkyl substitution in position 3, in particular a t-butyl substitution in position 3, and/or

- a substituted or unsubstituted $C_6$ to $C_{14}$ aryl or heteroaryl group in position 1, in particular a substituted or unsubstituted quinoline in position 1, more particularly a unsubstituted quinoline in position 1, and

wherein in particular $R^1$ is in ortho position in relation to the attachment position of the benzene moiety, wherein more particularly DA is in meta or para, in particular in para, position in relation to the attachment position of the benzene moiety to the parent moiety.

In some embodiments, $R^2$ is a substituted pyrazole connected to the benzene moiety of $R^2$ in position 5, and said pyrazole comprises a $C_1$-$C_6$ alkyl substitution in position 3, in particular t-butyl substitution in position 3, and a substituted or unsubstituted $C_6$ to $C_{14}$ aryl or heteroaryl group in position 1, in particular a substituted or unsubstituted quinoline in position 1, more particularly a unsubstituted quinoline in position 1, and

- n of $R^1_n$ is 0, 1, 2, 3 or 4, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, DA is -C(=O)NHR$^2$, -NHC(=O)R$^2$, -NHC(=O)NHR$^2$ -NHC(=S)NHR or -NHC(=O)NR$^2_2$, or
- n of $R^1_n$ is 0, 1, 2, 3 or 4, and each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, DA is -C(=O)NHR$^2$, -NHC(=O)R$^2$, -NHC(=O)NHR$^2$ or-NHC(=S)NHR$^2$, or
- n of $R^1_n$ is 0 or 1, and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, in particular $CH_3$, Cl or F, and DA is -C(=O)NHR$^2$, -NHC(=O)R$^2$, -NHC(=O)NHR$^2$, -NHC(=S)NHR$^2$, C(=O)NR$^2_2$ or-NHC(=O)NR$_2{}^2$, or
- n of $R^1_n$ is 0 or 1, and $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, in particular $CH_3$, Cl or F, DA is -C(=O)NHR$^2$, -NHC(=O)R$^2$, -NHC(=O)NHR$^2$ or-NHC(=S)NHR$^2$, or
- n of $R^1_n$ is 0 or 1, and $R^1$ is $CH_3$, Cl or F, and DA is -C(=O)NHR$^2$, -NHC(=O)R$^2$, -NHC(=O)NHR$^2$ or-NHC(=S)NHR$^2$, or
- n of $R^1_n$ is 0 or 1, and $R^1$ is $CH_3$, Cl or F, and DA is -NHC(=O)NHR$^2$, or
- n of $R^1_n$ is 0, and DA is C(=O)NHR$^2$, -NHC(=O)R$^2$ or -NHC(=O)NHR, or
- n of $R^1_n$ is 0, and DA is -NHC(=O)NHR$^2$, or
- n of $R^1_n$ is 1, and $R^1$ is $CH_3$, Cl or F, and DA is C(=O)NHR$^2$, -NHC(=O)R$^2$, or -NHC(=O)NHR$^2$, or
- n of $R^1_n$ is 1, and $R^1$ is $CH_3$, Cl or F, and DA is -NHC(=O)NHR$^2$, and

wherein in particular $R^1$ is in ortho position and/or DA is in meta or para, in particular in para, position in relation to the attachment position of the benzene moiety to the parent moiety.

In some embodiments, DA is -C(=O)NHR$^2$, -NHC(=O)R$^2$ or C(=O)NR$^2_2$ and DA is in meta position in relation to the attachment position of the benzene moiety; or DA is - NHC(=O)NHR$^2$ or -NHC(=S)NHR$^2$ and DA is in para position in relation to the attachment position of the benzene moiety.

Particular embodiments of this aspect of the invention are:

a. 2-amino-1-(3-(5-methyl-4H-1,2,4-triazol-3-yl)phenyl)-1H-pyrrolo[2,3-b]quinoxaline-3-carboxamide (compound 7),

b. 2-amino-1-(3-((3-(trifluoromethyl)phenyl)carbamoyl)phenyl)-1H-pyrrolo[2,3-b]quinoxaline-3-carboxamide (compound 10c),

c. 2-amino-1-(3-(3-(trifluoromethyl)benzamido)phenyl)-1H-pyrrolo[2,3-b]quinoxaline-3-carboxamide (compound 10d),

d. 2-amino-1-(3-(3-(3-(trifluoromethyl)phenyl)ureido)phenyl)-1H-pyrrolo[2,3-b]quinoxaline-3-carboxamide (compound 10f),

e. 2-amino-1-[2-methyl-5-[[3-(trifluoromethyl)benzoyl]amino]phenyl]pyrrolo[3,2-b]quinoxaline-3-carboxamide (compound 10g),

f. 2-amino-1-[2-fluoro-5-[[3-(trifluoromethyl)benzoyl]amino]phenyl]pyrrolo[3,2-b]quinoxaline-3-carboxamide (compound 10h),

g. 2-amino-1-[2-methyl-5-[[3-(trifluoromethyl)phenyl]carbamoyl]phenyl]pyrrolo[3,2-b]quinoxaline-3-carboxamide (compound 10i),

h. 2-amino-1-[2-fluoro-5-[[3-(trifluoromethyl)phenyl]carbamoyl]phenyl]pyrrolo[3,2-b]quinoxaline-3-carboxamide (compound 10j),

i. 2-amino-1-[3-[[3-(trifluoromethyl)phenyl]carbamothioylamino]phenyl]pyrrolo[3,2-b]quinoxaline-3-carboxamide (compound 10k),

j. 2-amino-1-[3-[[3-(4-methylimidazol-1-yl)-5-(trifluoromethyl)phenyl] carbamoyl]phenyl]pyrrolo[3,2-b]quinoxaline-3-carboxamide (compound 10l),

k. 2-amino-1-[3-[[3-(4-methylimidazol-1-yl)-5-(trifluoromethyl)benzoyl]amino]phenyl] pyrrolo[3,2-b]quinoxaline-3-carboxamide (compound 10m),

l. 2-amino-1-[3-[[5-tert-butyl-2-(6-quinolyl)pyrazol-3-yl]carbamoylamino]phenyl] pyrrolo[3,2-b]quinoxaline-3-carboxamide (compound 10n),

m. 2-amino-1-[3-[[5-tert-butyl-2-(p-tolyl)pyrazol-3-yl]carbamoylamino]phenyl] pyrrolo[3,2-b]quinoxaline-3-carboxamide (compound 10o),

n. 2-amino-1-(4-(3-(3-(trifluoromethyl)phenyl)ureido)phenyl)-1H-pyrrolo[2,3-b]quinoxaline-3-carboxamide (compound 10p),

In some embodiments, the compounds of the general formula (1) may be isolated in form of salts, in particular in form of pharmaceutically acceptable salts. The same applies to all of the before mentioned embodiments.

In some embodiments, the compounds of the general formula (1) may be isolated in form of a tautomer, a hydrate or a solvate.

Such salts are formed, for example, as acid addition salts, preferably with organic or inorganic acids, from compounds of the general formula (1) with a basic nitrogen atom, in particular the pharmaceutically acceptable salts are formed in such a way. Suitable inorganic acids are, without being limited to, halogen acids, such as hydrochloric acid, sulfuric acid, or phosphoric acid and the like. Suitable organic acids are, without being limited to, carboxylic, phosphonic, sulfonic or sulfamic acids and the like. Such organic acids may be, without being limited to, acetic acid, glycolic acid, lactic acid, malic acid, tartaric acid, or citric acid.

Salts may also be formed, for example, as salts with organic or inorganic bases, from compounds of the general formula (1) with a nitrogen atom bearing an acidic hydrogen. Examples of suitable cations are - without being limited to - sodium, potassium, calcium or magnesium cations, or cations of organic nitrogen bases, e.g. protonated mono-, di- or tri-(2-hydroxethyl)amine.

In view of the close relationship between the novel compounds in their free form and those in the form of their salts, any reference to the free compounds hereinbefore and hereinafter is to be understood as referring also to the corresponding salts, as appropriate and expedient. Likewise, in view of the close relationship between the novel compounds of the general formula (1) and their tautomers, any reference to the compounds of the general formula (1) is to be understood as referring also to the corresponding tautomers. The same applies to a hydrate or a solvate.

Another aspect of the invention relates to a pharmaceutical preparation comprising at least one compound according to one of the claims 1 to 12 as an active ingredient.

In some embodiments, the pharmaceutical preparation comprises at least one compound according to the invention as an active ingredient and at least one pharmaceutically acceptable carrier.

In some embodiments, the pharmaceutical preparation comprises at least one compound according to the invention in its free form as an active ingredient.

In some embodiments, the pharmaceutical preparation comprises at least one compound according to the invention in its free form as an active ingredient and at least one pharmaceutically acceptable carrier.

In some embodiments, the pharmaceutical preparation comprises at least one compound according to the invention in form of a salt, a tautomer, a pharmaceutically acceptable salt, a hydrate or a solvate.

In some embodiments, the pharmaceutical preparation comprises at least one compound according to the invention in form of a salt, a tautomer, a pharmaceutically acceptable salt, a hydrate or a solvate and at least one

pharmaceutically acceptable carrier.

Furthermore the invention relates to pharmaceutical preparations comprising at least one compound mentioned herein before as active ingredient, which can be used especially in the treatment of the diseases mentioned. The pharmaceutical preparations may be used in particular for a method for treatment of cancer or intraocular neovascular syndromes, more particularly for a method for treatment of cancer.

In some embodiments, the pharmaceutical preparations is for enteral administration, such as nasal, buccal, rectal or, especially, oral administration, and for parenteral administration, such as intravenous, intramuscular or subcutaneous administration, to warm-blooded animals, in particular humans, are especially preferred. The preparations comprise the active ingredient alone or, in particular, together with a pharmaceutically acceptable carrier. The dosage of the active ingredient depends upon the disease to be treated and upon the species, its age, weight, and individual condition, the individual pharmacokinetic data, and the mode of administration.

In some embodiments, a pharmaceutical preparation for the treatment of angiogenesis dependent cancers and intraocular neovascular syndromes of a warm-blooded animal, in particular a human requiring such treatment, comprises a compound of the general formula (1) as active ingredient in a quantity that is therapeutically active against the said diseases.

In some embodiments, the pharmaceutical preparations comprise from approximately 1% to approximately 95% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

In some embodiments, the pharmaceutical preparations of the present invention are prepared in a manner known *per se*, for example by means of conventional mixing, granulating, coating, dissolving or lyophilizing processes.

In some embodiments, the pharmaceutical preparations is in form of solutions of the active ingredient, and also suspensions or dispersions, especially isotonic aqueous solutions, dispersions or suspensions which, for example in the case of lyophilized preparations comprising the active ingredient alone or together with a carrier, for example mannitol, can be made up before use.

In some embodiments, the pharmaceutical preparations may be sterilized and/or may comprise excipients, for example preservatives, stabilizers, wetting agents and/or emulsifiers, solubilizers, salts for regulating osmotic pressure and/or buffers and are prepared in a manner known per se, for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80® (polyoxyethylene(20)sorbitan mono-oleate).

In some embodiments, the pharmaceutical preparation comprises suspensions in oil, which comprise as the oil component a vegetable, synthetic, or semi-synthetic oils customary for injection purposes.

In some embodiments, the pharmaceutical preparation comprises liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol.

In some embodiments, the pharmaceutical preparation comprises a mixtures of fatty acid esters, vegetable oils such as, without being limited to, cottonseed oil, almond oil, olive oil, castor oil, sesame oil, soybean oil and groundnut oil.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinyl pyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

In some embodiments, the pharmaceutical preparation is suitable for oral administration also include hard capsules consisting of gelatin, and also soft, sealed capsules consisting of gelatin and a plasticizer, such as glycerol or

sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxy ethylene sorbitan fatty acid ester type, may also be added.

In some embodiments, the pharmaceutical preparation is suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

In some embodiments, the pharmaceutical preparation is suitable for parenteral administration, aqueous solutions of an active ingredient in water-soluble form or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents. Solutions such as are used, for example, for parenteral administration can also be employed as infusion solutions.

Preferred preservatives are, for example, antioxidants, such as ascorbic acid, or microbicides, such as sorbic acid or benzoic acid.

A further aspect of the invention relates to a compound according to any of the claims 1 to 12 or a pharmaceutical preparation according to claim 13 for use in a method of treatment of disease.

The compounds of the general formula (1) have valuable pharmacological properties.

Compounds of the general formula (1) inhibit Ephrin receptor kinase, in particular EphB4 kinase or EphA8 kinase, are modulating angiogenesis, and are especially appropriate for the use against diseases or disorders such as colon cancers, angiogenesis-dependent cancers, intraocular neovascular syndromes and related diseases, e.g. psoriasis and rheumatoid arthritis (see e.g. Folkman et al., J. Biol. Chem. 267:10931-34 (1992); Klagsbrun et al., Annu. Rev. Physiol. 53:217-39 (1991); and Garner A., "Vascular diseases," In: Pathobiology of Ocular Disease. A Dynamic Approach, Garner A., Klintworth G K, eds., 2nd Edition (Marcel Dekker, NY, 1994), pp 1625-1710).

Angiogenesis-dependent cancers are, for example, so-called solid tumors, especially cancers of the gastrointestinal tract, the pancreas, breast, stomach, cervix, bladder, kidney, prostate, ovaries, endometrium, lung, brain, melanoma, Kaposi's sarcoma, squamous cell carcinoma of head and neck, malignant pleural mesotheriorna, lymphoma or multiple myeloma, also haemangioblastoma and haemangioma, and further liquid tumors, e.g. leukemias.

Intraocular neovascular syndromes are e.g. diabetic retinopathy, neovascular glaucoma, ischemic retinopathies and macular degeneration, e.g. age-related macular degeneration. Other angiogenesis related diseases are restenosis, e.g. stent-induced restenosis, Crohn's disease, and Hodgkin's disease.

Furthermore the invention relates to a method of treatment of angiogenesis dependent cancers and intraocular neovascular syndromes in a patient in need thereof, characterized in that a therapeutically effective amount of a compound of formula 1 as described hereinbefore as such or in form of a pharmaceutical preparation comprising it is administered to the patient in need thereof.

Another aspect of the invention relates to a compound according to any of the claims 1 to 12 or a pharmaceutical preparation according to claim 13 for use in a method for treatment of cancer or intraocular neovascular syndromes, in particular for use in a method for treatment of cancer. The compounds of the general formula 1 can be administered as such or especially in the form of pharmaceutical preparations, preferably in an amount effective against the said diseases, to a warm-blooded animal, for example a human, requiring such treatment. In the case of an individual having a bodyweight of about 70 kg the daily dose administered is from approximately 0.05 g to approximately 5 g, preferably from approximately 0.25 g to approximately 1.5 g, of a compound of the present invention.

**Short description of the Figures:**

Fig.1 shows the selectivity profile of compound 10c (A), 10d (B), 10n (C) and 10o (D) tested on a panel of 456 protein kinases at DiscoveRx. The dendrogram was obtained from KinomeScan using the KinomeTree software.

Fig.2 shows the selectivity profile of compound dasatinib as published by DiscoveRx at www.discoverx.com/tools-resources/interaction-maps;

Fig.3 shows the selectivity profile of erlotinib as published by DiscoveRx at www.discoverx.com/tools-resources/interaction-maps;

Fig.4 shows the selectivity profile of sunitinib as published by DiscoveRx at www.discoverx.com/tools-resources/interaction-maps;

Fig.5 shows the log base-10 values of $GI_{50}$, TGI and $LC_{50}$ (as defined and measured according to the Examples section hereafter) of compound 10n, as well as a graphical representation of the Z-score of each given value ($GI_{50}$, TGI, $LC_{50}$) for a given cell line relative to the mean and standard deviation of that value for all 60 cell lines of the NCI-60 panel of cancer cell-lines;

Fig.6 shows the log base-10 values of $GI_{50}$, TGI and $LC_{50}$ (as defined and measured according to the Examples section hereafter) of compound 10o, as well as a graphical representation of the Z-score of each given value ($GI_{50}$, TGI, $LC_{50}$) for a given cell line relative to the mean and standard deviation of that value for all 60 cell lines of the NCI-60 panel of cancer cell-lines.

**Examples**

General methods:

All reactions, unless otherwise stated, were carried out under a nitrogen atmosphere using standard Schlenk-techniques. All reagents were used as received unless otherwise noted. Solvents were purchased in the best quality available, degassed by purging thoroughly with nitrogen and dried over activated molecular sieves of appropriate size. Alternatively, they were purged with argon and passed through alumina columns in a solvent purification system (Innovative Technology). Reactions were monitored by thin layer chromatography (TLC) using Merck TLC silica gel 60 $F_{254}$. Flash column chromatography was performed over silica gel (230-400 mesh). NMR spectra were recorded on AV300, AV2 400 or AV2 500 MHz Bruker spectrometers. Chemical shifts are given in ppm. The spectra are calibrated to the residual $^1H$ and $^{13}C$ signals of the solvents. Multiplicities are abbreviated as follows: singlet (s), doublet (d), triplet (t), quartet (q), doublet-doublet (dd), quintet (quint), septet (sept), multiplet (m), and broad (br). 2,3-Dichloroquinoxaline (1), o-toluidine, 5-methoxy-2-methylaniline, 3-amino-4-methylbenzyl alcohol, 2,6-dimethylaniline, 2-chloro-6-methylaniline were purchased from Fluka.

High-resolution electrospray ionization mass spectrometry was performed on a Finnigan MAT 900 (Thermo Finnigan, San Jose, CA, USA) double-focusing magnetic sector mass spectrometer. Ten spectra were acquired. A mass accuracy ≤2 ppm was obtained in the peak matching acquisition mode by using a solution containing 2 μL PEG200, 2 μL PPG450, and 1.5 mg NaOAc (all obtained from Sigma-Aldrich, Buchs, Switzerland) dissolved in 100 mL MeOH (HPLC Supra grade, Scharlau, E-Barcelona) as internal standard.

General synthesis of compounds of the invention:

A reaction pathway to the intermediate 2a is depicted in scheme 1.

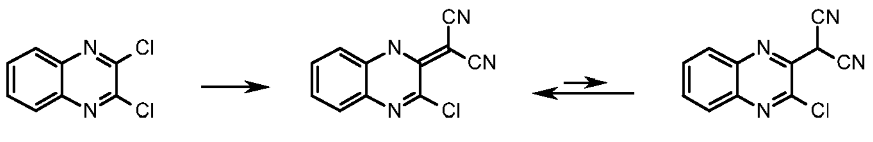

1 2a 2b

Scheme 1

The following is taken from (a) Pratt, E.F. et al, J Org Chem 1967, 32 (1):49-53, and (b) Obafemi, C.A., et al. Molecules 2004, 9 (4):223-231. To a solution of NaH (250 mg, 10.45 mmol) in anhydrous dimethoxyethane (20 mL) was carrefully added malononitrile (663 mg, 10.04 mmol). The reaction mixture was stirred at 25 °C for 30 min and 2,3-dichloroquinoxaline (1, 1 g, 5.02 mmol) was added. The resulting mixture was stirred at 25 °C for 3 h and then heated to reflux for one additional hour. The reaction mixture was cooled to 0 °C and 0.4 M HCl (30 mL) was slowly added. After stirring for 30 min at 0 °C, the formed precipitate was filtered off and recrystallized in EtOH to afford 2-(3-Chloroquinoxalin-2(1 H)-ylidene)malononitrile (2a) (845 mg, 74% yield).

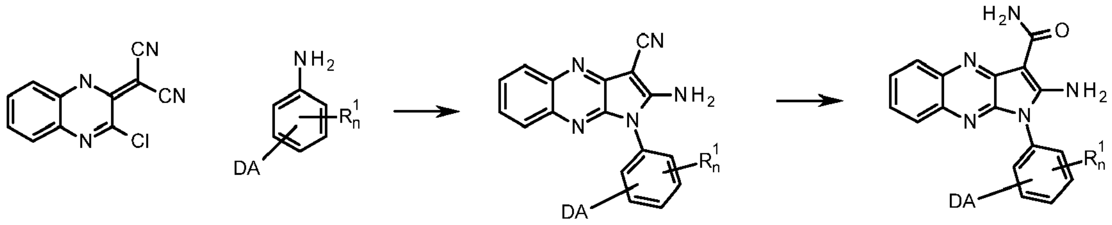

2a 3 9 10

Scheme 2

To a mixture of 2-(3-chloroquinoxalin-2-yl)malononitrile (2a, 1 eq) in a reaction solvent the primary amine (3, 1-4 eq) 8 was added. The reaction mixture was heated to a reaction temperature in the range of 80 to 130°C for 4 to 12 h, cooled, and the formed solid was filtered off and washed with a washing solution to afford the corresponding products in pure form or purified by flash column chromatography.

In some embodiments, the reaction solvent is selected from the group consisting of toluene, methanol, ethanol (EtOH), isopropanol, dimethylformamide and mixtures thereof, and is preferably EtOH, toluene or a mixture thereof. In some embodiments, the reaction solvent is a 1:1 EtOH:Toluene solution. In some embodiments, the reaction mixture is heated to a reaction temperature of 80 to 130 °C for 4 to 12 h. In some embodiments, the washing solution is selected from the group consisting of EtOH, ether. In some embodiments, the reaction product is concentrated under reduced pressure and purified by flash chromatography on silica gel.

Subsequently carbonitrile 9 (1 eq) was dissolved in concentrated sulfuric acid (0.15 M) stirred for 30 min at room temperature and poured into ice cold water. The solution was neutralized by the addition of 25% ammonium hydroxide solution and the formed solid was filtered off and optionally washed with a washing solution. Extraction afforded the corresponding products 10 in pure form, wherein optionally the extraction is carried out at a certain temperature. Optionally, the desired product may be washed with a washing solution. The organic layer was dried over $MgSO_4$, filtered, and concentrated under reduced pressure yields the product. Optional purification by column chromatography on silica gel may be applied. Additionally or alternatively the product may be re recrystallized.

In some embodiments, the basified water has a pH in the range of 5.5 to 7.5, and in particular of 6.0 to 7.0. In some embodiments, the water was basified with a solution of ammonium hydroxide (25%). In some embodiments, the reaction product is extracted with EtOAc and a saturated solution of $NaHCo_3$, EtOAc or acetone.

**Synthesis and Characterization**

The following compounds were prepared according to previously reported procedures:

Huang, W.S., et al., Synthesis-Stuttgart 2007, (14), 2121-2124:

2,3-dichloroquinoxaline (compound 1); 2-(3-Chloroquinoxalin-2(1 H)-ylidene)malononitrile (compound 2a), Yield 74 %, MS (ESI): *m/z:* calcd for $C_{11}H_5ClN_4Na^+$: 251.0, found: 250.9; 3-(4-Methyl-1 H-imidazol-1-yl)-5-(trifluoromethyl)phenylamine (compound 51), Yield: 73%; MS (ESI), *m/z:* calcd for $C_{11}H_{10}F_3N_3$, 242.1; found, 242.2.

Choi, H.G. et al., J. Med. Chem. 2010, 53 (15), 5439-5448:

3-(4-Methyl-1 H-imidazol-1-yl)-5-(trifluoromethyl)benzonitrile (a precursor for 6m), Yield: 71%; MS (ESI), *m/z*: calcd for $C_{12}H_9F_3N_3^+$, 252.07; found, 251.9; 3-(4-Methyl-1H-imidazol-1-yl)-5-(trifluoromethyl)benzoic acid (compound 6m), Yield: 93%; MS (ESI), *m/z:* calcd for $C_{12}H_8F_3N_2O_2$, 269.1; found, 268.9;

Flynn, D.L. et al., WO/2006/071940:

1-(quinolin-6-yl)hydrazine hydrochloride (precursor to 5n), Yield: 58%; MS (ESI), *m/z*: calcd for $C_9H_{10}N_3$ 160.1; found, 159.9; 3-tert-butyl-1-(quinolin-6-yl)-1H-pyrazol-5-amine (compound 5n), Yield: 42%; MS (ESI), *m/z:* calcd for $C_{16}H_{18}N_4$ 267.2; found, 267.0.

Regan, J., et al., *J Med Chem* 2002, *45* (14), 2994-3008:

3-amino-5-tert-butyl-2-p-tolyl-2H-pyrazole (compound 5o), Yield: 98%; MS (ESI), *m/z*: calcd for $C_{14}H_{20}N_3^+$ 230.17; found, 230.0.

Jung, M.E., et al. J Med Chem 2010, *53* (7), 2779-2796:

N-Boc-*p*-phenylenediamine (compound 3k), Yield: 97%; MS (ESI), *m/z:* calcd for $C_{11}H_{16}N_2NaO_2$ 231.11; found, 231.0.

Similar compounds may be produced in an analogue way to the hereine mentioned procedures and literature.

General procedure for the synthesis of amides

To a mixture of a primary amine (1 eq) in $CH_2Cl_2$ (0.5 M) was added triethylamine (1.05 eq) and benzoyl chloride (1.04 eq). The reaction was stirred at 25 °C for 15 h. The reaction mixture was poured into water, extracted with $CH_2Cl_2$, and the organic layer was dried over $MgSO_4$, filtered, and concentrated under reduced pressure. The residue was recrystallized from hexane/EtOAc to afford the desired product in pure form.

Examples are given further below. Should a different method be preferable a specified reaction pathway is provided.

Compound 4c: 3-nitro-N-(3-(trifluoromethyl)phenyl)benzamide

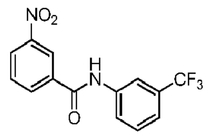

Yield: 79%; IR (film): $\tilde{\upsilon}$ = 3278, 3081, 1651, 1529, 1324, 1176, 1114, 694 $cm^{-1}$; MS (ESI): *m/z*: calcd for $C_{14}H_9F_3N_2O_3Na^+$: 333.2, found: 333.0.

Compound 4d: N-(3-nitrophenyl)-3-(trifluoromethyl)benzamide

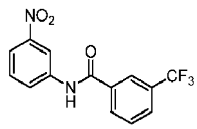

Yield: 54%; IR (film): $\tilde{\upsilon}$ = 3292, 1653, 1524, 1337, 1264, 1122, 1072 $cm^{-1}$; MS (ESI): *m/z*: calcd for $C_{14}H_9F_3N_2O_3Na^+$: 333.2, found: 333.0.

Compound 4g: N-(3-nitro-4-methylphenyl)-3-(trifluoromethyl)benzamide

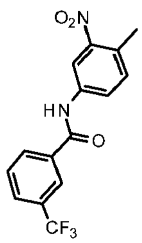

Yield: 99%; IR (neat): $\tilde{\upsilon}$ = 3325, 1656, 1617, 1587, 1524, 1443, 1386, 1338, 1319, 1308, 1260, 1238, 1160, 1124, 1111, 1069, 907, 889, 832, 821, 796, 751, 697, 670, 610, 561, 548, 534, 411 $cm^{-1}$; MS (ESI): *m*/*z*: calcd for $C_{15}H_{11}F_3N_2O_3^-$, 324.07; found, 323.0 (see Aquila, B., et al. WO2006/024834 A1, page 72).

Compound 4h: N-(3-nitro-4-fluorophenyl)-3-(trifluoromethyl)benzamide

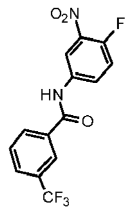

Yield: 48%;IR (neat): $\tilde{\upsilon}$ = 3266, 3145, 3078, 1655, 1616, 1532, 1406, 1346, 1325, 1255, 1170, 1121, 1070, 919, 889, 825, 814, 696, 665, 545, 497, 415 $cm^{-1}$; MS (ESI), *m*/*z*: calcd for $C_{14}H_8F_4N_2O_3^-$, 328.05; found, 326.9 (see Betebenner, D.A., et al., WO2007/076034 A2, page 138):

Compound 4i: 3-nitro-4-methyl-N-(3-(trifluoromethyl)phenyl) benzamide

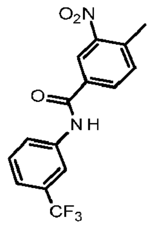

Yield: 78%;IR (neat): $\tilde{\upsilon}$ = 3303, 3078, 1656, 1616, 1567, 1530, 1494, 1348, 1321, 1270, 1229, 1166, 1152, 1116, 1096, 1069, 898, 882, 861, 842, 795, 740, 696, 661, 646, 632, 524 $cm^{-1}$; MS (ESI), *m*/*z*: calcd for $C_{15}H_{11}F_3N_2O_3^-$, 324.07; found, 323.0 (see Deak, H.L., et al., Bioorg Med Chem Lett 2008, 18 (3), 1172-1176).

Compound 4j: 3-nitro-4-fluoro-N-(3-(trifluoromethyl)phenyl) benzamide (unz-uh-23)

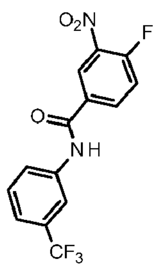

To a mixture of 4-fluoro-3-nitrobenzoic acid (1.0g, 5.40 mmol) in DCM (2.5mL) was added thionyl chloride (6.43g, 54.02 mmol) and it was refluxed for 3 h. The reaction mixture was concentrated under reduced pressure and a solution containing 3-(trifluoromethyl)aniline (0.87g, 5.40 mmol) and $Et_3N$ (1.13 mL, 8.10 mmol) in DCM (5 mL) was added. The reaction mixture was stirred at 25 °C for 12 hours, poured into water, filtered off and the precipitate was washed with water, $Et_2O$ and hexane affording 4j in pure form (1.37g, 77% yield).

IR (neat): $\tilde{\upsilon}$ = 3281, 3083, 1653, 1618, 1536, 1491, 1445, 1326, 1257, 1167, 1117, 1094, 1069, 899, 846, 799, 749, 672, 659, 628, 528 $cm^{-1}$. HRMS (ESI), *m*/*z*: calcd for $C_{14}H_7F_4N_2O_3$, 327.03983; found, 327.04006.

Compound 4l: 3-nitro-4-methyl-N-(3-(4-Methyl-1H-imidazol-1-yl)-5(trifluoromethyl)) benzamide

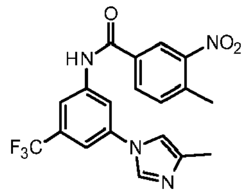

To a mixture of the aniline 5l (0.98g, 4.07 mmol) in THF (14 mL) was added DIPEA (1.38 mL, 8.14mmol) and 4-methyl-3-nitrobenzoyl chloride (0.71 mL, 4.88 mmol) at 0 °C. The reaction was stirred at 25 °C for 12 h.

The reaction mixture was poured into water, filtered off and washed with DCM and 1 M HCl affording the corresponding amide in pure form (0.94g, 57% yield). IR (neat): $\tilde{\upsilon}$ = 2991, 2762, 2620, 1675, 1612, 1569, 1529, 1473, 1444, 1380, 1346, 1299, 1245, 1230, 1167, 1121, 1093, 929, 887, 837, 797, 737, 715, 697, 666, 639, 583, 460, 406 $cm^{-1}$; HRMS (ESI), *m/z*: calcd for $C_{19}H_{16}F_3N_4O_3$ 405.11690; found, 405.11686.

Compound 4m: N-(3-nitro-4-methylphenyl)-3-(4-Methyl-1H-imidazol-1-yl)-5-(trifluoromethyl)benzamide

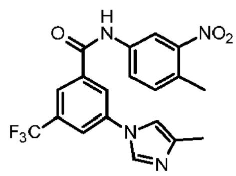

To a solution of the carboxylic acid 6m (1.0g, 3.91 mmol) in DMF (18mL), HOBt (0.59g, 4.69 mmol) and EDC (0.82mL, 4.69 mmol) was added 4-methyl-3-nitroaniline (0.71 g, 4.69 mmol) and the mixture was stirred at 25 °C for 12 hours.

The reaction mixture was concentrated under reduced pressure, redissolved in EtOAc and extracted with 0.5 M $NaHCO_3$ and water. The organic phase was dried over $MgSO_4$, concentrated under reduced pressure and the resulting solid was washed with DCM and hexane affording the desired amide in pure form (1.01g, 64% yield). IR (neat): $\tilde{\upsilon}$ = 3344, 1682, 1654, 1603, 1537, 1524, 1498, 1448, 1407, 1305, 1283, 1255, 1172, 1121, 1103, 1079, 1001, 904, 877, 829, 812, 751, 739, 690, 671, 617, 503, 418 $cm^{-1}$; MS (ESI), *m/z*: calcd for $C_{19}H_{16}F_3N_4O_3^+$, 405.11690; found, 405.11716.

General procedure for the synthesis of ureas

To a mixture of aniline (e.g. 5n, 5o)(1 eq) in $CH_2Cl_2$ (0.3 M) was added the isocyanate (1 eq). The reaction mixture was stirred at 25 °C for 1-3 d, and the resulting precipitate was filtered off and washed with DCM and hexane affording the desired compound in pure form.

Compound 4f: 1-(3-nitrophenyl)-3-(3-(trifluoromethyl)phenyl)urea

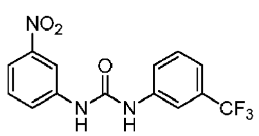

Yield: 92%; IR (film): $\tilde{\upsilon}$ = 3373, 3082, 1692, 1533, 1337, 1117, 733 $cm^{-1}$; MS (ESI): *m/z*: calcd for $C_{14}H_{10}F_3N_3O_3Na^+$: 348.2, found: 348.2.

Compound 4n: 1-(3-tert-butyl-1-(quinolin-6-yl)-1H-pyrazol)-3-(4-nitrophenyl)urea

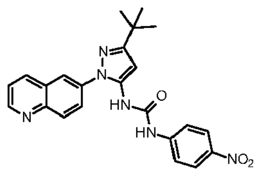

Yield: 99 %; IR (neat): $\tilde{\upsilon}$ = 3356, 2960, 1732, 1539, 1499, 1379, 1329, 1301, 1272, 1241, 1175, 1112, 1020, 985, 850, 832, 792, 751, 689, 630, 604, 497, 425 $cm^{-1}$; HRMS (ESI), *m*/*z*: calcd for $C_{23}H_{23}N_6O_3^+$ 431.18262; found, 431.18254.

Compound 4o: 1-(4-tert-butyl-2-p-tolyl-2H-pyrazole)-3-(4-nitrophenyl)urea

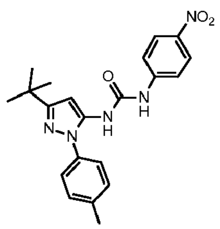

Yield: 97%; IR (neat): $\tilde{\upsilon}$ = 3339, 2960, 1732, 1543, 1498, 1328, 1302, 1269, 1188, 1174, 1137, 1110, 1014, 996, 852, 822, 750, 689, 499, 440 $cm^{-1}$; HRMS (ESI), *m*/*z*: calcd for $C_{21}H_{24}N_5O_3^+$ 394.18737; found, 394.18744.

Compound 4p: 1-(4-nitrophenyl)-3-(3-(trifluoromethyl)phenyl)urea

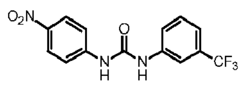

Yield: 60%; IR (neat): $\tilde{\upsilon}$ =3356, 3315, 1719, 1658, 1617, 1556, 1494, 1484, 1446, 1325, 1305, 1234, 1175, 1163, 1109, 1070, 931, 884, 849, 796, 751, 697, 653, 495 $cm^{-1}$. MS (ESI), *m*/*z*: calcd for $C_{14}H_9F_3N_3O_3^-$, 324.07; found, 324.0 (see Oguro, Y., et al., Bioorgan Med Chem 2010, 18 (20), 7260-7273).

General procedure A for the reduction of nitro phenyls

To a mixture of nitrophenyl (compound 4) (1 eq) in EtOH (0.1 M) was added $SnCl_2$ (5 eq). The reaction mixture was heated to 100 °C for 2 h, cooled, and concentrated under reduced pressure. A solution of EtOAc/NaOH (1 M) was added to the residue, and the resulting precipitate was filtered off. The organic layer was washed with brine, dried over $MgSO_4$, filtered, and concentrated under reduced pressure to afford the desired product in pure form.

Compound 3c: 3-amino-N-(3-(trifluoromethyl)phenyl)benzamide

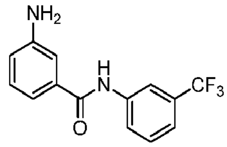

Yield: 77%; IR (film): $\tilde{\upsilon}$ = 3457, 1655, 1539, 1439, 1329, 1120, 793 $cm^{-1}$; MS (ESI): *m/z:* calcd for $C_{14}H_{11}F_3N_2ONa^+$: 303.2, found: 303.0.

Compound 3d: N-(3-aminophenyl)-3-(trifluoromethyl)benzamide

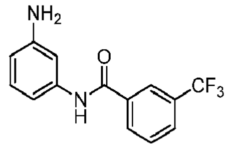

Yield: 81%; IR (film): $\tilde{\upsilon}$ = 3399, 3246, 1603, 1322, 1263, 1128, 696 $cm^{-1}$; MS (ESI): *m/z*: calcd for $C_{14}H_{11}F_3N_2ONa^+$: 303.2, found: 303.0.

Compound 3f:1-(3-aminophenyl)-3-(3-(trifluoromethyl)phenyl)urea

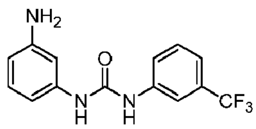

Yield: 92%; IR (film): $\tilde{\upsilon}$ = 3306, 1633, 1557, 1333, 1110, 769 $cm^{-1}$; MS (ESI): *m/z*: calcd for $C_{14}H_{12}F_3N_3ONa^+$: 318.3, found: 318.1.

General procedure B for the reduction of nitro phenyls

To a solution of nitrophenyl (compound 4) (1 eq) in MeOH (0.3 M) was added 10% Pd/C (10% wt). The reaction mixture was stirred at 25 °C under a hydrogen balloon for 4-12 hours. The reaction mixture was filtered through a pad of celite and concentrated under reduced pressure obtaining the corresponding anilines in pure form. This method or an adapted method thereof was used to obtain the following intermediates. Further Literature reference can be found with the specific compound.

Compound 3g: N-(3-amino-4-methylphenyl)-3-(trifluoromethyl)benzamide

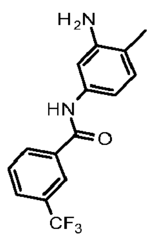

Yield: 77%; IR (neat): $\tilde{\upsilon}$ = 3490, 3385, 3271, 1647, 1624, 1615, 1602, 1544, 1518, 1414, 1381, 1326, 1268, 1163, 1111, 1071, 998, 923, 857, 812, 723, 696, 684, 652, 616, 461, 414 $cm^{-1}$; MS (ESI), *m/z*: calcd for $C_{15}H_{13}F_3N_2O^-$

, 294.10 (see Choi, H.G., et al., J Med Chem 2010, 53 (15), 5439-5448).

<u>Compound 3h:</u> N-(3-amino-4-fluorophenyl)-3-(trifluoromethyl)benzamide

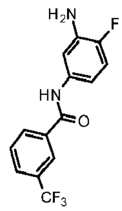

Yield: 99%; IR (neat): $\tilde{\upsilon}$ = 3427, 3300, 1666, 1624, 1555, 1516, 1416, 1328, 1255, 1168, 1107, 1068, 924, 847, 801, 772, 741, 690, 575, 450, 419 $cm^{-1}$; HRMS (ESI), *m/z*: calcd for $C_{14}H_9F_4N_2O^-$, 297.06565; found, 297.06543.

<u>Compound 3i:</u> 3-amino-4-methyl-N-(3-(trifluoromethyl)phenyl)benzamide

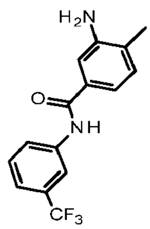

Yield: 99%; IR (neat): $\tilde{\upsilon}$ = 3369, 3320, 1665, 1644, 1576, 1551, 1441, 1331, 1318, 1259, 1224, 1175, 1162, 1119, 1105, 1066, 1000, 919, 895, 796, 743, 697, 662, 449, 433 $cm^{-1}$; MS (ESI), *m/z*: calcd for $C_{15}H_{13}F_3N_2O^-$, 294.10; found, 293.0 (see Deak, H.L., et al., Bioorg Med Chem Lett 2008, 18 (3), 1172-1176).

<u>Compound 3j:</u> 3-amino-4-fluoro-N-(3-(trifluoromethyl)phenyl) benzamide

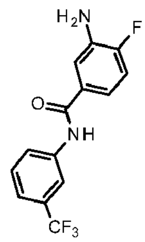

Yield: 92%; IR (neat): $\tilde{\upsilon}$ = 3406, 3253, 3206, 3088, 1661, 1604, 1561, 1511, 1444, 1431, 1336, 1324, 1282, 1261, 1198, 1179, 1162,1101, 1066, 919, 899, 870, 849, 791, 771, 752, 724, 694, 660, 642, 556, 450 $cm^{-1}$; HRMS (ESI), *m/z*: calcd for $C_{14}H_9F_4N_2O^-$, 297.06565; found, 297.06555.

<u>Compound 3l:</u> 3-amino-4-methyl-N-(3-(4-Methyl-1 H-imidazol-1-yl)-5(trifluoromethyl)) benzamide

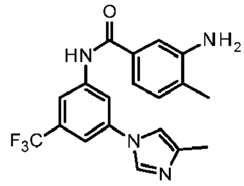

Yield: 52%; IR (neat): $\tilde{\upsilon}$ = 3328, 3229, 2795, 1667, 1613, 1541, 1508, 1480, 1441, 1398, 1381, 1304, 1231, 1201, 1171, 1123, 1088, 998, 929, 876, 850, 823, 745, 707, 693, 634, 539, 424 $cm^{-1}$; HRMS (ESI), *m/z*: calcd for $C_{19}H_{18}F_3N_4O^+$ 375.14272; found, 375.14317.

Compound 3m: N-(3-amino-4-methylphenyl)-3-(4-Methyl-1 H-imidazol-1-yl)-5(trifluoromethyl)benzamide

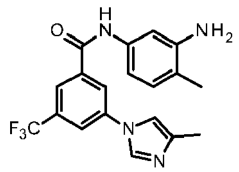

Yield: 44%; IR (neat): $\tilde{\upsilon}$ = 3328, 3119, 2969, 1656, 1598, 1543, 1499, 1454, 1426, 1398, 1377, 1330, 1316, 1284, 1253, 1164, 1115, 1078, 1003, 881, 867, 796, 724, 691, 451, 430, 419, 402 $cm^{-1}$; MS (ESI), *m/z*: calcd for $C_{19}H_{18}F_3N_4O^+$, 375.14272; found, 375.14295.

Compound 3n: 1-(3-tert-butyl-1-(quinolin-6-yl)-1H-pyrazol)-3-(4-aminophenyl)urea

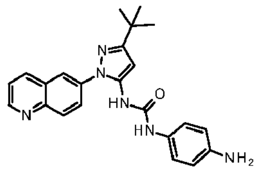

Yield: 89%; IR (neat): $\tilde{\upsilon}$ = 3331, 2959, 1716, 1627, 1599, 1546, 1512, 1428, 1376, 1314, 1239, 1195, 1174, 1133, 987, 904, 829, 799, 781, 635, 612, 523, 481, 473 $cm^{-1}$; HRMS (ESI), *m/z*: calcd for $C_{23}H_{25}N_6O^+$ 401.20844; found, 401.20888.

Compound 3o: 1-(4-tert-butyl-2-p-tolyl-2H-pyrazole)-3-(4-aminophenyl)urea

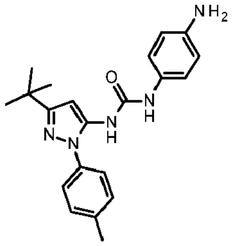

Yield: 98%; IR (neat): $\tilde{\upsilon}$ = 3349, 3276, 2959, 1643, 1614, 1591, 1552, 1513, 1370, 1295, 1215, 1170, 1119, 1014, 989, 817, 801, 654, 570, 522, 501, 414 $cm^{-1}$; HRMS (ESI), *m/z*: calcd for $C_{21}H_{26}N_5O^+$ 364.21319; found, 364.21317.

Compound 3p: 1-(4-aminophenyl)-3-(3-(trifluoromethyl)phenyl)urea

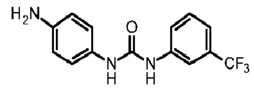

Yield: 69%; IR (neat): $\tilde{\upsilon}$ =3304, 2873, 1599, 1656, 1615, 1556, 1515, 1493, 1448, 1424, 1329, 1311, 1230, 1215, 1164, 1119, 1096, 1068, 884, 794, 741, 699, 655, 512 $cm^{-1}$; MS (ESI), *m/z*: calcd for $C_{14}H_{13}F_3N_3O^+$, 296.26 ; found, 296.0 (see Oguro, Y., et al., Bioorgan Med Chem 2010, 18 (20), 7260-7273).

General procedure A for the cyclization of 2-(3-chloroquinoxalin-2-yl)malononitrile

To a mixture of 2-(3-chloroquinoxalin-2-yl)malononitrile (1 eq) in a 1 : 1 EtOH - toluene solution (0.09 M) was added the primary amine (compounds 3) (2 eq in general, 1 eq for 2-Amino-1-(N-Boc-p-phenylamine)-1H-pyrrolo[2,3-b]quinoxaline-3-carbonitrile and 4 eq for 2-amino-1-(3-(5-methyl-4H-1,2,4-triazol-3-yl)phenyl)-1H-pyrrolo[2,3-b]quinoxaline-3-carbonitrile, whose precursor 3-(5-methyl-4H-1,2,4-triazol-3-yl)phenyl amine hydrate was purchased from ABCR). The reaction mixture was heated to 130 °C for 4-12 h, cooled, and the formed solid was filtered off and washed with $Et_2O$ and EtOH to obtain the desired compounds in pure form.

Compound 9a: 2-amino-1-(3-(5-methyl-4H-1,2,4-triazol-3-yl)phenyl)-1H-pyrrolo[2,3-b]quinoxaline-3-carbonitrile

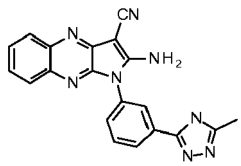

Yield: 44 %, IR (film): $\tilde{\upsilon}$ = 3452, 3312, 3173, 2219, 1659, 1561, 1466, 1410, 1045, 740 $cm^{-1}$; HRMS (ESI): *m/z*: calcd for $C_{20}H_{14}N_8H^+$: 367.1414, found: 367.1417.

Compound 9c: 3-(2-amino-3-cyano-1H-pyrrolo[2,3-b]quinoxalin-1-yl)-N-(3-(trifluoromethyl)phenyl)benzamide,

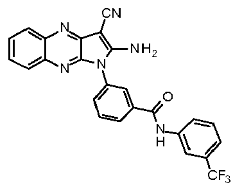

Yield: 31%; IR (film): $\tilde{\upsilon}$ = 3346, 3184, 2211, 1660, 1561, 1443, 1331, 1121, 596 $cm^{-1}$; HRMS (ESI): *m/z:* calcd for $C_{25}H_{15}F_3N_6OH^+$: 473.1332, found: 473.1334.

Compound 9d: N-(3-(2-amino-3-cyano-1H-pyrrolo[2,3-b]quinoxalin-1-yl)phenyl)-3-(trifluoromethyl)benzamide

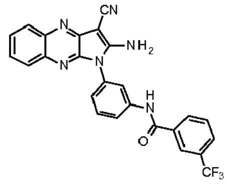

Yield: 78%; IR (film): $\tilde{\upsilon}$ = 3326, 3155, 2210, 1663, 1561, 1426, 1115, 660 $cm^{-1}$; HRMS (ESI): *m/z:* calcd for $C_{25}H_{15}F_3N_6ONa^+$: 495.1152, found: 495.1146.

Compound 9f: 1-(3-(2-amino-3-cyano-1H-pyrrolo[2,3-b]quinoxalin-1-yl)phenyl)-3-(3-(trifluoromethyl)phenyl)urea

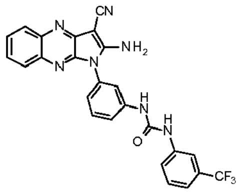

Yield: 54%; IR (film): $\tilde{\upsilon}$ = 3322, 3157, 2208, 1663, 1560, 1446, 1320, 1163, 1117, 754 $cm^{-1}$; HRMS (ESI): *m/z:* calcd for $C_{25}H_{16}F_3N_7OH^+$: 488.1441, found: 488.1442.

Compound 9h: N-(3-(2-am!no-3-cyano-1H-pyrrolo [2,3-b] quinoxalin-1-yl)-4-fluorophenyl)-3-(trifluoromethyl)benzamide,

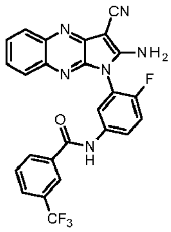

Yield: 57%; IR (neat): $\tilde{\upsilon}$ =3338, 3076, 2230, 1649, 1624, 1552, 1511, 1471, 1390, 1329, 1253, 1223, 1120, 1073, 917, 834, 809, 755, 693, 658, 596, 510, 419 $cm^{-1}$; HRMS (ESI), *m/z:* calcd for $C_{25}H_{15}F_4N_6O^+$, 491.12380; found, 491.12401.

Compound 9j: 3-(2-amino-3-cyano-1H-pyrrolo[2,3-b]quinoxalin-1-yl)-4-fluoro-N-(3-(trifluoromethyl)phenyl)benzamide

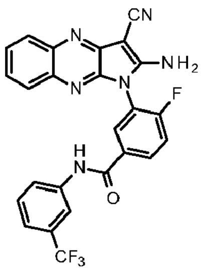

Yield: 65%; IR (neat): $\tilde{\upsilon}$ =3258, 3121, 2226, 1884, 1686, 1652, 1618, 1581, 1563, 1515, 1493, 1471, 1389, 1330, 1277, 1212, 1172, 1120, 1095, 1073, 887, 844, 796, 761, 748, 694, 594, 517, 510, 479, 443 $cm^{-1}$; HRMS (ESI),

*m/z:* calcd for $C_{25}H_{15}F_4N_6O^+$, 491.12380; found, 491.12436.

The not pure compounds were purified by preparative thin layer chromatography (hex/EtOAc,1:1)

Compound 9g: N-(3-(2-amino-3-cyano-1H-pyrrolo[2,3-b]quinoxalin-1-yl)-4-methyl phenyl)-3-(trifluoromethyl)benzamide

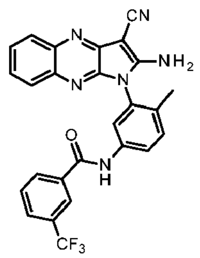

Yield: 30%; IR (neat): $\tilde{\upsilon}$ =3326, 3166, 2212, 1656, 1596, 1565, 1508, 1485, 1445, 1391, 1326, 1309, 1256, 1203, 1166, 1124, 1092, 1073, 1037, 817, 755, 693, 650, 599, 477, 411 $cm^{-1}$; HRMS (ESI), *m/z:* calcd for $C_{26}H_{18}F_3N_6O^+$, 487.14887; found,487.14919.

Compound 9i: 3-(2-amino-3-cyano-1H-pyrrolo[2,3-b]quinoxalin-1-yl)-4-methyl-N-(3-(trifluoromethyl)phenyl)benzamide

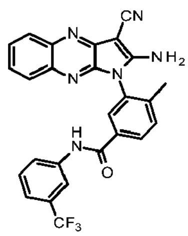

Yield: 33%; IR (neat): $\tilde{\upsilon}$ =3323, 3130, 2922, 2360, 2205, 1651, 1555, 1443, 1332, 1260, 1165, 1121, 1069, 796, 759, 696, 667, 659, 598, 418 $cm^{-1}$; HRMS (ESI), *m/z:* calcd for $C_{26}H_{18}F_3N_6O^+$, 487.14887; found,487.14932 or flash column chromatography (hex/EtOAc,1:1)

Compound 9k: 2-Amino-1-(N-Boc-p-phenylamine)-1H-pyrrolo[2,3-b]quinoxaline-3-carbonitrile

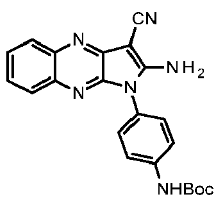

Yield:46%; IR (neat): $\tilde{\upsilon}$ = 3349, 3256, 3160, 2214, 1700, 1671, 1592, 1565, 1502, 1482, 1443, 1413, 1394, 1369, 1314, 1238, 1214, 1166, 1058, 1028, 1020, 840, 752, 624, 599, 528, 418 $cm^{-1}$; HRMS (ESI), m/z: calcd for $C_{22}H_{21}N_6O_2^+$ 401.17205; found, 401.17200.

General procedure B for the cyclization of 2-(3-chloroquinoxalin-2-yl)malononitrile

To a mixture of 2-(3-chloroquinoxalin-2-yl)malononitrile (1 eq) in DMF (0.1 M) was added the primary amine (1.2 eq). The reaction mixture was heated to 80 °C for 12 h, concentrated and purified by flash column chromatography

(hexane/EtOAc, 1:1 to EtOAc/MeOH/$Et_3N$, 90:9:1). This method was used to obtain intermediates of Compound 9p: 1-(4-(2-amino-3-cyano-1H-pyrrolo[2,3-b]quinoxalin-1-yl)phenyl)-3-(3-(trifluoromethyl)phenyl)urea

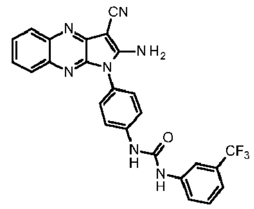

Yield: 61%; IR (neat): $\tilde{\upsilon}$ = 3282, 3066, 2597, 2225, 1655, 1622, 1602, 1551, 1514, 1493, 1446, 1336, 1313, 1232, 1201, 1174, 1166, 1120, 1069, 834, 793, 757, 696, 668, 659, 596, 510, 483,474, 420 $cm^{-1}$; HRMS (ESI), *m/z:* calcd for $C_{25}H_{17}F_3N_7O^+$, 488.14412; found, 488.14409.

Compound 9n: 1-(4-(2-amino-3-cyano-1H-pyrrolo[2,3-b]quinoxalin-1-yl)phenyl)-3-(5-tert-butyl-2-quinolin-6-yl-2H-pyrazol)urea

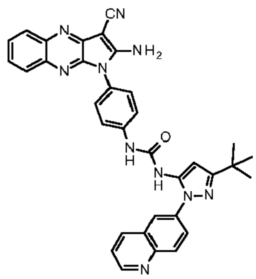

Yield: 62%; IR (neat): $\tilde{\upsilon}$ = 3312, 3169, 2957, 2923, 2856, 2208, 1712, 1653, 1548, 1506, 1438, 1377, 1361, 1309, 1263, 1241, 1201, 1137, 1123, 1022, 986, 899, 885, 836, 756, 599, 529, 417 $cm^{1}$; HRMS (ESI), *m/z:* calcd for $C_{34}H_{29}N_{10}O^+$ 593.25203; found, 593.25208.

Compound 9o: 1-(3-(2-amino-3-cyano-1H-pyrrolo[2,3-b]quinoxalin-1-yl)phenyl)-3-(4-tert-butyl-2-p-tolyl-2H-pyrazole)urea

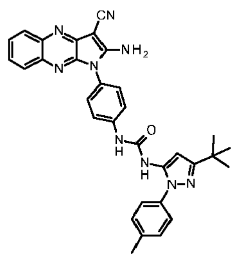

Yield: 55%; IR (neat): $\tilde{\upsilon}$ = 3275, 2959, 2863, 1642, 1552, 1514, 1441, 1369, 1295, 1210, 1172, 1120, 1034, 1014, 990, 817, 800, 756, 666, 598, 570, 527, 520, 504, 449, 404 $cm^{-1}$; HRMS (ESI), m/z: calcd for $C_{32}H_{30}N_9O^+$ 556.25678; found, 556.25682.

General procedure C for the cyclization of 2-(3-chloroquinoxalin-2-yl)malononitrile

To a mixture of 2-(3-chloroquinoxalin-2-yl)malononitrile (1 eq) in DMF (0.1 M) was added the imidazol derivative (1.0 eq). The reaction mixture was heated to 80 °C for 12 h, concentrated and purified by flash column chromatography (EtOAc/MeOH, 9:1). This method was used to obtain intermediates of

Compound 9l: 3-(2-amino-3-cyano-1H-pyrrolo[2,3-b]quinoxalin-1-yl)-4-methyl-N-(4-methyl-1H-imidazol-1-yl)-5-(trifluoromethyl)benzamide)

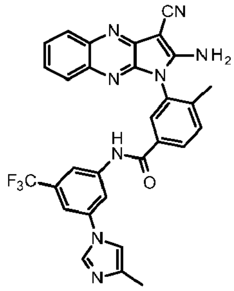

Yield: 21%; IR (neat): $\tilde{\upsilon}$ = 3303, 2924, 2855, 2202, 1683, 1653, 1614, 1565, 1499, 1479, 1444, 1407, 1325, 1309, 1269, 1241, 1227, 1193, 1170, 1123, 1103, 1082, 1021, 1008, 930, 863, 808, 758, 750, 694, 671, 627, 597, 523, 473, 446, 413, 402 $cm^{-1}$; HRMS (ESI), *m/z:* calcd for $C_{30}H_{22}F_3N_8O^+$ 567.18632; found, 567.18610.

Compound 9m: N-(3-(2-amino-3-cyano-1H-pyrrolo[2,3-b]quinoxalin-1-yl)-4-methylphenyl)-3-((4-methyl-1 H-imidazol-1-yl)-5-(trifluoromethyl)benzamide)

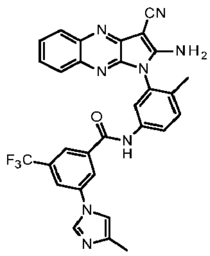

Yield: 24%; IR (neat): $\tilde{\upsilon}$ = 3299, 3066, 2925, 2204, 1651, 1603, 1565, 1538, 1498, 1463, 1393, 1378, 1317, 1281, 1251, 1176, 1125, 1080, 1011, 880, 817, 755, 691, 664, 598, 446 $cm^{-1}$; H RMS (ESI), *m/z:* calcd for $C_{30}H_{22}F_3N_8O^+$, 567.18632; found, 567.18616.

General procedure for the hydrolysis of carbonitriles

A solution of the carbonitrile (compounds 9) (1 eq) in sulfuric acid (0.15 M) was stirred for 30 min at 25 °C. The reaction was poured into ice cold water, basified with a solution of ammonium hydroxide (25%) and the formed solid was filtered off and washed with cold water. In the case of 2-amino-1-(3-(5-methyl-4H-1,2,4-triazol-3-yl)phenyl)-1H-pyrrolo[2,3-b]quinoxaline-3-carboxamide (7) the residue was redissolved in EtOAc and extracted with a saturated solution of $NaHCO_3$ affording the corresponding product in pure form.

Compound 10a: 2-amino-1-(3-(5-methyl-4H-1,2,4-triazol-3-yl)phenyl)-1H-pyrrolo[2,3-b]quinoxaline-3-carboxamide

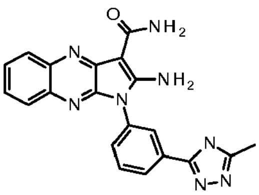

Yield: 13%; mp 320-322 °C; IR (film): $\tilde{\upsilon}$ = 3370, 3302, 1592, 1524, 1129, 754 $cm^{-1}$; HRMS (ESI): *m*/*z:* calcd for $C_{20}H_{16}N_8O)H^+$: 385.1520, found: 385.1522.

Compound 10c: 2-amino-1-(3-((3-(trifluoromethyl)phenyl)carbamoyl)phenyl)-1H-pyrrolo[2,3-b]quinoxaline-3-carboxamide

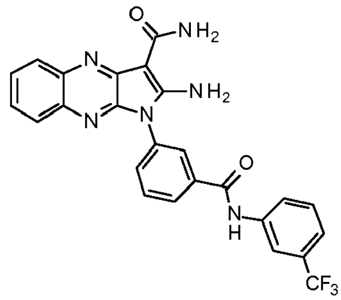

Yield: 61%; mp 312-314 °C; $^1H$ NMR (500 MHz, DMSO-$d_6$): $\delta$ = 10.62 (s, 1 H), 8.24-8.22 (m, 5H), 8.08 (d, *J* = 8.4 Hz, 1 H), 7.96 (dd, *J* = 8.3 Hz, *J* = 1.0 Hz, 1 H), 7.89-7.84 (m, 2H), 7.80-7.78 (m, 2H), 7.64-7.57 (m, 2H), 7.49-7.46 (m, 2H), 7.39 (s, 1 H); IR (film): $\tilde{\upsilon}$ = 3436, 3272, 1607, 1511, 1334, 1112, 751 $cm^{-1}$; HRMS (ESI): *m*/*z:* calcd for $C_{25}H_{17}F_3N_6O_2H^+$: 491.1438, found: 491.1440.

Compound 10d: 2-amino-1-(3-(3-(trifluoromethyl)benzamido)phenyl)-1H-pyrrolo[2,3-b]quinoxaline-3-carboxamide

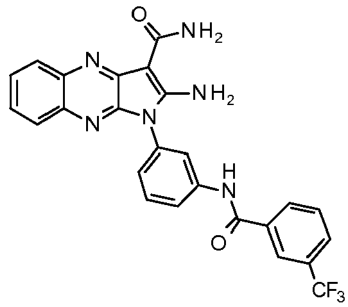

Yield: 33%; mp 302-304 °C; $^1H$ NMR (500 MHz, DMSO-$d_6$): $\delta$ = 10.77 (s, 1 H), 8.32 (s, 1 H), 8.29 (d, *J* = 7.9 Hz, 1 H), 8.15-8.05 (m, 3H), 8.00-7.95 (m, 3H), 7.83-7.79 (m, 3H), 7.67 (t, *J* = 8.1 Hz, 1 H), 7.60-7.57 (m, 1 H), 7.49-7.46 (m, 1 H), 7.37-7-36 (m, 2H); IR (film): $\tilde{\upsilon}$ = 3452, 3362, 3275, 1639, 1490, 1330, 1171, 757 $cm^{-1}$; HRMS (ESI): *m*/*z:* calcd for $C_{25}H_{17}F_3N_6O_2H^+$: 491.1438, found: 491.1443.

Compound 10f: 2-amino-1-(3-(3-(3-(trifluoromethyl)phenyl)ureido)phenyl)-1H-pyrrolo[2,3-b]quinoxaline-3-carboxamide

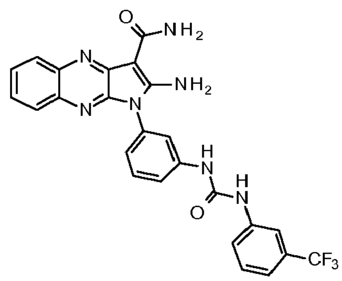

Yield: 15%; mp 303-305 °C; $^1$H NMR (400 MHz, DMSO-$d_6$): δ = 9.45 (s, 1H), 9.40 (s, 1H), 8.12 (s, 2H), 8.02 (s, 1H), 7.95 (d, *J* = 7.5 Hz, 1 H), 7.80-7.78 (m, 2H), 7.72-7.68 (m, 2H), 7.60-7.45 (m, 5H), 7.36 (s, 1 H), 7.31 (d, *J* = 7.6 Hz, 1 H), 7.20 (d, *J* = 8.4 Hz, 1 H); IR (film): $\tilde{\upsilon}$ = 3504, 3405, 3292, 1637, 1601, 1566, 1331, 1108, 754 cm$^{-1}$; HRMS (ESI): *m/z:* calcd for $C_{25}H_{18}F_3N_7O_2H^+$: 506.1547, found: 506.1547.

Compound 10g: 2-amino-1-(2-methyl-5-(3-(trifluoromethyl)benzamido)phenyl)-1H-pyrrolo[2,3-b]quinoxaline-3-carboxamide

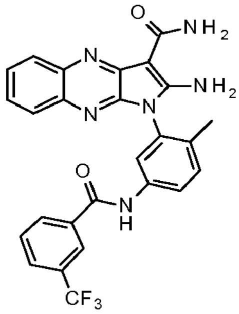

Yield: 70%; $^1$H NMR (500 MHz, DMSO-$d_6$): δ=10.69 (s, 1 H), 8.31 (s, 1 H), 8.27 (d, *J* = 7.9 Hz, 1 H), 7.99 - 7.95 (m, 3H), 7.87 (s, 1 H), 7.81 - 7.77 (m, 3H), 7.58 (t, *J* = 7.5 Hz, 1 H), 7.54 (d, *J* = 8.4 Hz, 1 H), 7.47 (t, *J* = 7.8 Hz, 1 H), 7.39 (s, 1 H), 2.01 (s, 3H); IR (neat): $\tilde{\upsilon}$ = 3301, 3220, 3063, 1637, 1592, 1570, 1509, 1440, 1417, 1332, 1251, 1169, 1121, 1091, 1072, 822, 799, 757, 694, 616, 597, 471, 439, 429 cm$^{-1}$; HRMS (ESI), *m/z:* calcd for $C_{26}H_{20}F_3N_6O^+$, 505.15981; found, 505.15943.

Compound 10h: 2-amino-1-(2-fluoro-5-(3-(trifluoromethyl)benzamido)phenyl)-1H-pyrrolo[2,3-b]quinoxaline-3-carboxamide (unz-uh-31)

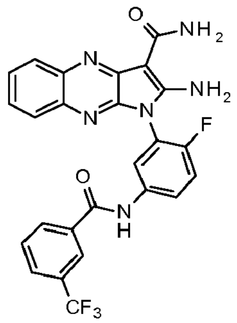

Yield: 68%; $^1$H NMR (500 MHz, DMSO-$d_6$): δ=10.80 (s, 1 H), 8.33 - 8.28 (m, 4H), 8.08 - 8.07 (m, 2H), 7.99 (d, *J* = 7.5 Hz, 1 H), 7.96 (d, *J* = 8.3 Hz, 1 H), 7.83 - 7.77 (m, 3H), 7.63 - 7.58 (m, 2H), 7.49 (t, *J* = 7.5 Hz, 1 H), 7.42 (s, 1 H); IR (neat): $\tilde{\upsilon}$ =3293, 3201, 3073, 1638, 1571, 1462, 1417, 1401, 1332, 1250, 1168, 1122, 1071, 1031, 808, 758, 695, 597, 474, 454, 433 cm$^{-1}$; HRMS (ESI), *m/z:* calcd for $C_{25}H_{17}F_4N_6O_2^+$, 509.13436 ; found, 509.13434.

Compound 10i: 2-amino-1-(2-methyl-(5-((3-(trifluoromethyl)phenyl)carbamoyl)phenyl)-1H-pyrrolo[2,3-b]quinoxaline-3-carboxamide

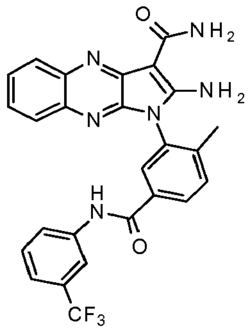

Yield: 75%;$^1$H NMR (500 MHz, DMSO-$d_6$): δ=10.53 (s, 1H), 8.21 - 8.15 (m, 5H), 8.08 (d, *J* = 8.0 Hz, 1 H), 7.96 (d, *J* = 7.4 Hz, 1 H), 7.78 - 7.77 (m, 2H), 7.73 (d, *J* = 8.1 Hz, 1 H), 7.62 - 7.57 (m, 2H), 7.49 - 7.45 (m, 2H), 7.40 (s, 1 H), 2.13 (s, 3H); IR (neat): =3289, 3213, 2926, 2158, 1638, 1570, 1525, 1492, 1443, 1332, 1167, 1121, 1070, 796, 755, 697, 595, 473, 459, 446, 432 $cm^{-1}$; HRMS (ESI), *m/z:* calcd for $C_{26}H_{20}F_3N_6O^+$, 505.15943; found, 505.15937.

Compound 10j: 2-amino-1-(2-fluoro-(5-((3-(trifluoromethyl)phenyl)carbamoyl)phenyl)-1H-pyrrolo[2,3-b]quinoxaline-3-carboxamide

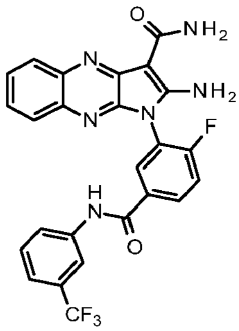

Yield: 99%; $^1$H NMR (500 MHz, DMSO-$d_6$): δ= 10.64 (s, 1 H), 8.39 - 8.34 (m, 3H), 8.21 (s, 2H), 8.07 (d, *J* = 7.8 Hz, 1 H), 7.97 (d, *J* = 8.1 Hz, 1 H), 7.80 - 7.74 (m, 3H), 7.64 - 7.59 (m, 2H), 7.50 - 7.47 (m, 2H), 7.43 (s, 1H); IR (neat): $\tilde{\upsilon}$ =3292, 3207, 3070, 1641, 1571, 1514, 1491, 1444, 1332, 1275, 1167, 1116, 1094, 1070, 797, 754, 697, 614, 597, 481, 419 $cm^{-1}$. HRMS (ESI), *m/z:* calcd for $C_{25}H_{17}F_4N_6O_2^+$, 509.13436; found, 509.13434.

Compound 10l: 2-amino-1-(2-methyl-5-((3-(4-Methyl-1H-imidazol-1-yl)-(5-(trifluoromethyl) phenyl) carbamoyl)phenyl)-1H-pyrrolo[2,3-b]quinoxaline-3-carboxamide

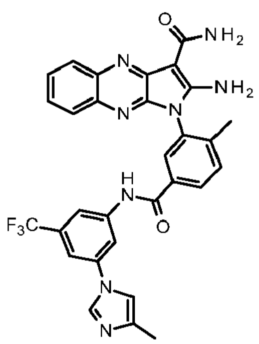

Yield: 85%; $^1$H NMR (500 MHz, DMSO-$d_6$): δ= 10.66 (s, 1 H), 8.27 (s, 1 H), 8.22 - 8.15 (m, 5H), 7.97 (d, *J* = 8.4 Hz, 1 H), 7.78 - 7.73 (m, 4H), 7.59 (t, *J* = 7.6 Hz, 1 H), 7.49 - 7.46 (m, 2H), 7.40 (s, 1 H), 7.09 (br, 2H), 2.16 (s, 3H),

2.15 (s, 3H); IR (neat): $\tilde{\upsilon}$ = 3303, 2923, 2202, 1683, 1653, 1614, 1565, 1499, 1478, 1444, 1325, 1309, 1268, 1241, 1226, 1193, 1170, 1123, 1103, 1082, 1021, 1008, 929, 863, 807, 758, 750, 694, 670, 627, 597, 579, 523, 470, 452, 439, 411 $cm^{-1}$; HRMS (ESI), *m/z:* calcd for $C_{30}H_{24}F_3N_8O_2^+$ 585.19688; found, 585.19698.

Compound 10m: 2-amino-1-(2-methyl-5-((4-Methyl-1H-imidazol-1-yl)-5-(trifluoromethyl)benzamido)phenyl)-1H-pyrrolo[2,3-b]quinoxaline-3-carboxamide

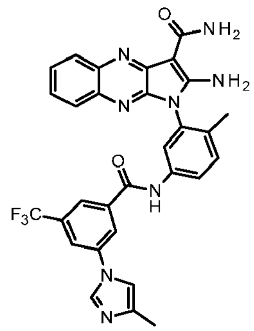

Yield: 90%; $^1H$ NMR (500 MHz, DMSO-$d_6$): δ= 10.71 (s, 1 H), 8.45 (s, 1 H), 8.39 (br, 1 H), 8.25 (s, 1 H), 8.18 (s, 1 H), 8.13 (br, 1 H), 8.00 (d, *J* = 8.1 Hz, 1 H), 7.96 (d, *J* = 8.1 Hz, 1 H), 7.84 (s, 1 H), 7.79 - 7.77 (m, 2H), 7.69 (s, 1 H), 7.60 - 7.56 (m, 2H), 7.47 (t, *J* = 7.7 Hz, 1 H), 7.38 (s, 1 H), 2.18 (s, 3H), 2.03 (s, 3H); IR (neat): $\tilde{\upsilon}$ = 3048, 1643, 1571, 1509, 1450, 1376, 1303, 1250, 1174, 1090, 884, 837, 756, 691, 614, 598, 460 $cm^{-1}$; MS (ESI), *m/z:* calcd for $C_{30}H_{24}F_3N_8O_2^+$, 585.19688; found, 585.19636.

Compound 10n: 2-amino-1-(4-(3-(5-tert-butyl-2-quinolin-6-yl-2H-pyrazol)ureido)phenyl)-1H-pyrrolo[2,3-b]quinoxaline-3-carboxamide

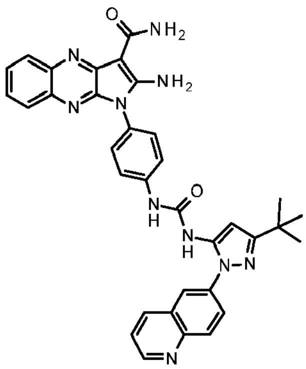

Yield 87%; 1 H-NMR (400 MHz, DMSO-$d_6$): δ= 9.32 (s, 1 H), 8.97 (dd, *J* = 4.1, 1.3 Hz, 1 H), 8.68 (s, 1 H), 8.51 - 8.47 (m, 1 H), 8.22 - 8.15 (m, 2H), 7.98 (dd, *J* = 9.0, 2.2 Hz, 2H), 7.93 (dd, *J* = 8.3, 1.1 Hz, 1 H), 7.78 - 7.72 (m, 2H), 7.69 - 7.60 (m, 3H), 7.60 - 7.53 (m, 1 H), 7.50 - 7.41 (m, 3H), 7.32, (br, 1 H), 6.49 (s, 1 H), 1.34 (s, 9H); IR (neat): Ũ = 3203, 2958, 1706, 1644, 1506, 1463, 1413, 1363, 1312, 1193, 1080, 1029, 986, 835, 758, 614, 597, 573, 527, 515, 478, 471, 447, 436, 418, 405 $cm^{-1}$; HRMS (ESI), *m/z:* calcd for $C_{34}H_{31}N_{10}O_2^+$ 611.26260; found, 611.26242.

Compound 100: 2-amino-1-(4-(3-(4-tert-butyl-2-p-tolyl-2H-pyrazole)ureido)phenyl)-1H-pyrrolo[2,3-b]quinoxaline-3-carboxamide

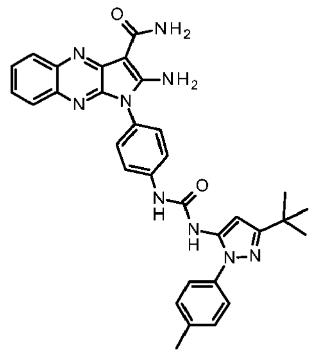

Yield 90%; $^1$H NMR (500 MHz, DMSO-$d_6$): δ= 9.36 (s, 1 H), 7.93 (d, *J* = 8.7 Hz, 1 H), 7.77 - 7.76 (m, 2H), 7.66 (d, *J* = 9.1 Hz, 2H), 7.57 (t, *J* = 6.7 Hz, 1 H), 7.47-7.42 (m, 5H), 7.36 - 7.33 (m, 3H), 6.39 (s, 1 H), 2.39 (s, 3H), 1.29 (s, 9H); IR (neat): $\tilde{\upsilon}$ = 3220, 2959, 1643, 1515, 1413, 1367, 1313, 1194, 1087, 987, 823, 756, 613, 598, 524, 475, 459, 436 cm$^{-1}$; HRMS (ESI), *m/z:* calcd for $C_{32}H_{32}N_9O_2^+$ 574.26735; found, 574.26729.

Compound 10p: 2-amino-1-(4-(3-(3-(trifluoromethyl)phenyl)ureido)phenyl)-1H-pyrrolo[2,3-b]quinoxaline-3-carboxamide

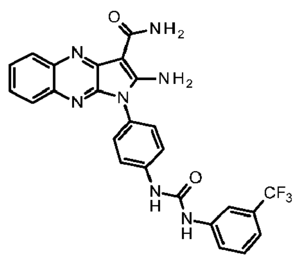

Yield: 99%; $^1$H NMR (400 MHz, DMSO-$d_6$): δ= 9.61 (br, 1 H), 8.07 (s, 1 H), 7.92 (d, *J* = 7.8 Hz, 1 H), 7.78 - 7.74 (m, 4H), 7.68 (d, *J* = 7.5 Hz, 1 H), 7.57 - 7.51 (m, 2H), 7.49 - 7.44 (m, 3H), 7.32 (s, 1 H), 7.30 (s, 1 H); IR (neat): $\tilde{\upsilon}$ = 3305, 1637, 1567, 1515, 1493, 1446, 1336, 1314, 1232, 1174, 1120, 1096, 1069, 794, 756, 698, 662, 614, 597, 528, 516, 473, 457, 421, 413 cm$^{-1}$; HRMS (ESI), m/z: calcd for $C_{25}H_{19}F_3N_7O_2^+$, 506.15468; found, 506.15493.

Compound 10e: 2-amino-1-(4-aminophenyl)pyrrolo[3,2-b]quinoxaline-3-carboxamide

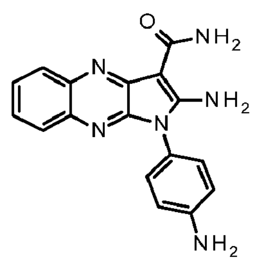

Yield: 86%; $^1$H-NMR (400 MHz, DMSO-$d_6$): δ= 7.92 (d, *J* = 7.8 Hz, 1 H), 7.77 - 7.70 (m, 2H), 7.56 (t, *J* = 7.5 Hz, 1 H), 7.46 (t, *J* = 7.3 Hz, 1 H), 7.30 (br, 1 H), 7.19 (d, J = 8.6 Hz, 2H), 6.81 (d, *J* = 8.5 Hz, 2H); IR (neat): $\tilde{\upsilon}$ = 3337, 3186, 2957, 1635, 1567, 1519, 1444, 1414, 1365, 1315, 1297, 1265, 1237, 1193, 1167, 1058, 1030, 832, 797, 753, 707, 612, 596, 518, 474, 461, 447, 433, 421 cm$^{-1}$; HRMS (ESI), *m/z:* calcd for $C_{17}H_{15}N_6O^+$ 319.13019; found, 319.13059.

Compound 10k: 2-amino-1-(4-(3-(3-(trifluoromethyl)phenyl)thioureido)phenyl)-1H-pyrrolo[2,3-b]quinoxaline-3-carboxamide

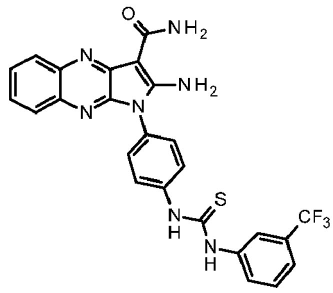

To a mixture of the aniline 10e (10mg, 0.031 mmol) in DMF (0.1 mL) was added 3-(trifluoromethyl)phenyl isothiocyanate (3.7 μL, 0.024 mmol). The reaction mixture was stirred at 25 °C for 12 hours, and the resulting precipitate was filtered off and washed with DCM and hexane, affording the desired thiourea in pure form (8.6mg, 69% yield).

Yield: 69%; $^1H$-NMR (400 MHz, DMSO-$d_6$): δ= 10.29 (s, 1 H), 10.18 (s, 1 H), 8.02 (s, 1 H), 7.95 (d, *J* = 7.4 Hz, 1 H), 7.83 - 7.78 (m, 5H), 7.62 - 7.56 (m, 4H), 7.51 - 7.45 (m, 2H), 7.35 (s, 1 H); IR (neat): $\tilde{\upsilon}$ = 3302, 3193, 2917, 2848, 1639, 1602, 1515, 1448, 1330, 1164, 1120, 1095, 1069, 797, 758, 696, 667, 596, 521, 475, 464, 449, 439 $cm^{-1}$; HRMS (ESI), *m*/*z:* calcd for $C_{25}H_{19}F_3N_7OS^+$ 522.13184; found, 522.13236.

**In vitro evaluation:**

In the following, compounds 17 and 87 (in the tables) correspond to prior art compounds 1 and 2 of Figure 5 in Zhao H. and Huang D. "Hydrogen Bonding Penalty upon Ligand Binding", PLoS ONE, 2011, Volume 6, Issue 6. These were purchased from InterBioScreen.

Cell culture and $GI_{50}$ determination:

MDA-MB-231 (breast cancer) and KM12 (colon cancer) cells obtained from the UZH Cancer Institute, HOP-92 (lung cancer) cells purchased from the National Cancer Institute (NCI) and A498 (kidney cancer) cells obtained from Prof. Krek (Institute of Cell Biology, ETH Zurich) were cultured in Dulbelcco's modified Eagle's medium (DMEM) supplemented with 10% (v/v) fetal bovine serum. K562 leukemia cells obtained from Dr. Silvio Hemmi (Institute of Molecular Life Sciences, UZH) were cultured using RPMI medium supplemented with 5% (v/v) fetal bovine serum. All the media was additionally supplemented with 100 units/mL of penicillin, 100μg/mL of streptomycin, 4.5 g/L glucose, 0.11g/L sodium pyrubate and 2mM glutamine and the cells were grown at 37°C in 5% $CO_2$ atmosphere with 80% relative humidity.

MDA-MB-231, HT-29, KM12, HOP-92 and A498 cells were plated at 10,000 cells per well (100 μL per well) in 96-well culture dishes and allowed to incubate for 24 h. The old media was removed, the cells were washed with PBS (phosphate-buffered saline) and fresh media was added. A 5 mM solution of inhibitor (in 100% DMSO) was serially diluted in the culture media (12 different concentrations were used) and allowed to incubate for 72 h (MDA-MB-231) or 48h (A498). Control cells were treated with the same DMSO concentrations. After the incubation period the media was once more removed and the cells were washed with PBS to then be incubated with fresh media containing 86nM resazurin. After 3 hours, the fluorescence was quantified using a fluorescence microplate reader (Biotek, FLx800™) at the respective excitation and emission wavelength of 560 and 590 nm. Resazurin becomes fluorescent under mitochondrial reduction and its emission directly correlates with the metabolic stability of the cells. The measured fluorescence values were corrected from the control samples containing DMSO and $IC_{50}$ values were determined by plotting the fraction of metabolically active cells against the log of the drug concentration. All measurements were performed at least in triplicate (see table 1).

In the case of K-562 leukemia cells, they were seeded at a density of 20,000 cells per well in 100μL of RMPI media in a 96-well microtiter plates. After 24 hours, 12.5 μL of a 10 fold concentrated drug or DMSO solution in RMPI media was added in every well. After 48 hour incubation, cell viability was studied by measuring the ability of the cells to process resazurin. Resazurin was added to every well to obtain a final concentration of 86nM, and after 3 hours, the fluorescence was quantified using a fluorescence microplate reader (Biotek, FLx800™) at the respective excitation and emission wavelength of 560 and 590nm as described above. All measurements were performed at least in triplicate (see table 1 b).

**Table 1a:** $GI_{50}$ MDA-MB-231

| Compound no. | $GI_{50}$ (μmol/l) |
|---|---|
| 10c | 10.80 |
| 10d | 3.09 |
| 10p | 10.26 |
| 10g | 3.88 |
| 10h | 1.44 |
| 10i | 1.32 |
| 10j | 2.69 |
| 10k | 9.63 |
| 10l | 3.05 |
| 10m | 1.93 |
| 10n | 0.21 |
| 10o | 0.98 |

**Table 1b:** $GI_{50}$ K-562

| Compound no. | $GI_{50}$ (μmol/l) |
|---|---|
| 10c | 0.82 |
| 10d | 1.52 |
| 10p | 5.45 |
| 10g | 0.73 |
| 10h | 0.37 |
| 10i | 0.036 |
| 10j | 0.081 |
| 10k | 5.07 |
| 10l | 0.029 |
| 10m | 0.03 |
| 10n | 0.003 |
| 10o | 0.02 |
| Prior Art 17 | 35.63 |
| Prior Art 87 | 73.07 |

**Table 1c:** $GI_{50}$ A-498

| Compound no. | $GI_{50}$ (μmol/l) | Compound no. | $GI_{50}$ (μmol/l) |
|---|---|---|---|
| 10c | 10.60 | 10k | 8.73 |
| 10d | 13.36 | 10l | 5.71 |
| 10p | 10.84 | 10m | 4.01 |
| 10g | 2.50 | 10n | 0.73 |
| 10h | 1.88 | 10o | 1.00 |
| 10i | 2.07 | Prior Art 17 | 49.96 |
| 10j | 2.44 | Prior Art 87 | 99.64 |

**Table 1d:** $GI_{50}$ HT-29

| Compound no. | $GI_{50}$ (μmol/l) | Compound no. | $GI_{50}$ (μmol/l) |
|---|---|---|---|
| 10c | 23.1 | 10j | 2.97 |

(continued)

| **Compound no.** | **$GI_{50}$ (μmol/l)** | **Compound no.** | **$GI_{50}$ (μmol/l)** |
|---|---|---|---|
| 10d | 29.4 | 10k | 16.3 |
| 10p | 18.0 | 10l | 4.36 |
| 10g | 5.87 | 10m | 13.4 |
| 10h | 1.80 | 10n | 1.25 |
| 10i | 2.82 | 10o | 0.78 |

**Table 1e:** $GI_{50}$ KM12

| **Compound no.** | **$GI_{50}$ (μmol/l)** | **Compound no.** | **$GI_{50}$ (μmol/l)** |
|---|---|---|---|
| 10c | 2.78 | 10j | 1.87 |
| 10d | 3.98 | 10k | 2.52 |
| 10p | 0.67 | 10l | 2.84 |
| 10g | 0.80 | 10m | 2.54 |
| 10h | 2.16 | 10n | 0.15 |
| 10i | 1.57 | 10o | 0.21 |

**Table 1f:** $GI_{50}$ HOP-92

| **Compound no.** | **$GI_{50}$ (μmol/l)** | **Compound no.** | **$GI_{50}$ (μmol/l)** |
|---|---|---|---|
| 10c | 5.13 | 10j | 2.12 |
| 10d | 4.65 | 10k | 4.25 |
| 10p | 10.0 | 10l | 3.01 |
| 10g | 4.23 | 10m | 5.12 |
| 10h | 2.06 | 10n | 0.45 |
| 10i | 1.92 | 10o | 1.48 |

Surface Plasmon Resonance (SPR) determination of Kd:

The EphA3-compound dissociation constant (Kd) in Table 2 were obtained following the experimental protocol of Jecklin M.C., et al (Journal of Molecular Recognition 22(4), 319-329), subject to the following modifications:

1) EphA3 protein was purified as per the protocol given in the "Experimental Section" of Lafleur K., Dong J., Huang D., Caflisch A. and C. Nevado (Journal of Medicinal Chemistry, 2013; 56(1):84-96), under the header "Protein Expression and Purification". Protein concentration was determined using a NanoDrop 1000 from Thermo Scientific (Witec AG, Littau LU, Switzerland).

2) Under "Instrumentation and reagents": 20 mM PBS pH 7.4, 150 mM NaCl was used as running buffer.

3) Under "Enzyme immobilization": Following activation of flow cells, 300 μg/ml of EphA3 in immobilization buffer was injected over a 2 minute time period. Residual activated groups on the surface were then blocked by a 7 minute injection of 1 M ethanolamine, pH 8.5. A total of 6000 response units (RU) of EphA3 were immobilized.

4) Under "Analyte injection method and order": 5 mM DMSO solutions of each of the compounds in Table 2 were serially diluted by the aforementioned running buffer to form suspensions with the following compound concentrations: 18 nM, 55 nM, 166 nM and 500 nM. Each suspension was then analyzed, commencing from the lowest and proceeding to the highest concentration, and then run in duplicate from the highest to the lowest concentration. During each binding cycle, the compound suspension was injected during a time period of 6 minutes at a flow rate of 5 μl/min and dissociation was monitored for 900 s.

**Table 2:** SPR Kd (nmol/l)

| Compound no. | SPR kd (nmol/l) |
|---|---|
| 10c | 3.7 |
| 10d | 40.09 |

Cellular phosphorylation assays: The cellular phosphorylation experiments were performed at ProQinase GmbH. Mouse embryonic fibroblast cells, stably transfected to overexpress full-length human EphB4, were plated at $4\times10^4$ cells/well in DMEM supplemented with 10% FCS in 48-well culture dishes. Medium was replaced by DMEM without FCS before test compounds prediluted in 100% DMSO were added (final DMSO concentration of 1 %). After incubation for 90 min at 37 °C, cells were stimulated for 2 h at 4 °C using murine ephrinB2-Fc at a final concentration of 2 μg/ml. Quantification of EphB4 phosphorylation was assessed in a 96-well plate via sandwich ELISA using a myc capture antibody and an antiphosphotyrosine detection antibody.

**Table 4:** Cell $IC_{50}$ (μmol/l) in cells overexpressing EphB4

| Compound no. | cell $IC_{50}$ (nmol/l) | Compound no. | Cell $IC_{50}$ (nmol/l) |
|---|---|---|---|
| 10c | 16 | 10j | 24 |
| 10d | 20 | 10l | 270 |
| 10p | 89 | 10m | 170 |
| 10g | 14 | 10n | 1100 |
| 10h | 15 | 10o | 370 |
| 10k | 560 | Prior Art 17 | 640 |
| 10i | 21 | | |

EphA3 expression and purification: A clone of EphA3 kinase domain (residues: 606-947) was obtained from Prof. Sirano DhePaganon's group (Choi, Y., et al., Bioorg Med Chem Lett 2009, 19 (15), 4467-4470) and expressed in *Escherichia coli* strain BL21 (DE3). Cells expressing EphA3 were induced with a 1 mM solution of isopropyl-beta-D-thiogalactopyranoside (IPTG) for 12h at 15°C.

Cell pellets were resuspended in buffer A (50mM Tris, pH 8.0 and 100mM NaCl, supplemented with protease inhibitors) and lysed by sonication. After centrifugation at 15,000 rpm for 1 h, the soluble fraction of EphA3 was purified using HisTrap Q HP columns (GE healthcare), followed by gel filtration chromatography (Superdex75; GE healthcare). The appropriate fractions were combined and concentrated to ~10 mg/mL using Amicon filter devices (10kDa as cutoff) in a storage solution (100mM sodium chloride and 10mM Tris-HCl pH 8.0, 5% glycerol). The resulting solution was aliquoted and stored at -80°C for further usage.

Differential Scanning Fluorimetry: To a 2 μM solution of EphA3 in 10mM Tris HCl buffer (pH 8.0) containing 100mM NaCl, 5X SYPRO Orange fluorescent dye together with the test compounds prediluted in DMSO at a concentration of 20μM (1.4% final DMSO concentration). A final volume of 40μL was added per well in a 96 well plate and incubated at room temperature for 1 h. The plates were centrifuged for 3 min at 450 rcf. The temperature gradient was performed in the range of 25°C-75°C (50 acquisitions per degree at a ramp rate of 4.4°C/s) using a LightCycler 480 Real-Time PCR System. The detection of protein unfolding was performed with an excitation wavelength of 488 nm and an emission of 580 nm. All measurements were performed in triplicate.

**Table 5:** Delta Tm with EphA3

| Compound no. | Delta Tm | Compound no. | Delta Tm |
|---|---|---|---|
| 10c | 9.1 | 10k | 9.9 |
| 10d | 7.6 | 10l | 16.2 |
| 10p | 14.3 | 10m | 15.8 |
| 10g | 12.3 | 10n | 19.3 |
| 10h | 10.9 | 10o | 17.3 |
| 10i | 14.41 | Prior Art 17 | 1.6 |
| 10j | 13.0 | Prior Art 87 | 0.7 |

**In vivo evaluation:**

Evaluation of the in vivo pharmacokinetics of a subset of compounds was performed through analysis of compound concentration in the blood plasma of male CD-1 (ICD) mice (each weighing 20-30g) over a 24 hour period (at time points of 3, 10, 30, 60, 120, 240, 360 and1440 minutes) following a single oral (PO) and intravenous (IV, in tail vein) administration of a compound dose corresponding to 5 mg of compound/kg of mouse body weight (PO) and 1 mg of compound/kg of mouse body weight (IV). For IV dosing, each compound was formulated as a clear solution in DMSO/Solutol® HS 15/phosphate-buffered saline, pH 7.4 (PBS) (5/5/90, v/v/v). For PO dosing, compounds were formulated as a homogeneous suspension in DMSO/1% methylcellulose (5/95, v/v).

At the relevant time point, blood samples (300-400 μL) were collected from the mice under general inhalant anaesthesia by cardiac puncture and stored on ice in 0.6 mL polypropylene heparin-lithium-coated tubes. Within 1 hour of collection, each tube was centrifuged at 2500 G for 15 minutes at 4°C and harvested plasma stored at -20°C.

Compound concentration in plasma at each time point was determined following acetonitrile precipitation of each sample followed by HPLC-MS analysis performed in triplicates, i.e., based on the mean of the compound concentration at that time point in blood samples of three mice.

In the following table 6:

T1/2 is the terminal elimination half-life following IV and PO dosing (obtained from non-compartmental analysis (NCA) of the plasma data using WinNonLin). Tmax is the time following PO administration at which the maximum concentration is observed (obtained from non-compartmental analysis (NCA) of the plasma data using WinNonLin). Cmax corresponds to the concentration observed at Tmax following PO administration (obtained from non-compartmental analysis (NCA) of the plasma data using WinNonLin). Bioavailability was calculated as the ratio of: [AUClast(PO)*Dose(IV)] / [AUClast(IV)*Dose(PO)] where AUClast is the area under the curve (estimated by summing incremental areas of a series of trapezoids) of a plot of compound concentration versus time, up to the time of the last observation that is greater than the limit of quantification.

**Table 6:** In vivo pharmacokinetics (min: minutes)

| Compound no. | T1/2 (min, IV) | T1/2 (min, PO) | Cmax (ng/mL, PO) | Tmax (min, PO) | Bio-availability (%) |
|---|---|---|---|---|---|
| 10c | 66 | 167 | 164 | 60 | 30 |
| 10d | 71 | 297 | 45 | 120 | 10 |

Selectivity profiles:

Compounds 10c, 10d, 10n and 10o show selective binding affinity (defined as less than 10 percent of control binding to the kinase in presence of 1 μM concentration of the compound relative to control binding in absence of the compound) relative to one or more of the following protein kinases: ABL1 (E255K)-phosphorylated, ABL1 (F3171)-nonphosphorylated, ABL1 (F317L)-phosphorylated, ABL1 (F317L)-nonphosphorylated, ABL1 (H396P)-phosphorylated, ABL1 (Y253F)-phosphorylated, ABL1 (H396P)-nonphosphorylated, ABL1 (Q252H)-nonphosphorylated, ABL1 (Q252H)-phosphorylated, ABL1-phosphorylated, ABL1-nonphosphorylated, ABL2, ACVR2B, AXL, BLK, BMPR1 B, BMX, BRAF(V600E), BRK, BTK, CDK11, CDK2, CDK3, CDK5, CDK7, CDK8, CIT, CLK1, CLK4, CSF1 R, CSK, DDR1, DDR2, EGFR(L858R), EGFR(L861Q), EGFR(T790M), EPHA1, EPHA2, EPHA4, EPHA5, EPHA8, EPHB1, EPHB2, EPHB3, EPHB4, EPHB6, ERBB2, FAK, FES, FGFR1, FGR, FLT3, FLT3(ITD), FLT3(K663Q), FLT3(N841I), FRK, FYN, HASPIN, HCK, INSRR; ITK, KIT, KIT(A829P), KIT(D816V), KIT(V559D), KIT(V559D, V654A), KIT(V559D, V670I), LCK, LIMK1, LIMK2, LOK, LTK, LYN, MEK5, MERTK, MET(Y1235D), MST1, NEK9, PCTK2, PCTK3, PDGFRA, PDGFRB, PFTAIRE2, PFTK1, PIP5K1C, PKMYT1, PYK2, RET, RET(M918T), RET(V804L), RET(V804M), RSK3(Kinase domain 1, N-terminal), SLK, SRC, TAK1, TEC, TESK1, TIE1, TIE2, TGFBR1, TNK1, TNNI3K, TRKA, TRKB, TRKC, TXK, YES, ZAK.

The selectivity profiles of compounds 10c, 10d, 10n and 10o are depicted in Figure 1. Figure 2 (dasatinib), Figure 3 (erlotinib) and Figure 4 (sunitinib) show the selectivity of FDA approved compounds for comparison. The legend applicable to Fig. 2-4 is in Fig. 2.

The selectivity profiling of compounds 10c, 10d, 10n and 10o were performed at DiscoveRx in a library of 456 kinases at a concentration of 1 μM. Briefly, kinases were mostly expressed as fusion proteins to T7 phase and grown in 24-well blocks in *E. Coli* (derived from the BL21 strain). The rest of the kinases were expressed in HEK-293 cells and tagged with DNA for qPCR detection. Affinity resins were generated by mixing streptavidin-coated magnetic beads with biotinylated small molecule ligands for 30 minutes at 25 °C, followed by biotin and blocking buffer addition (SeaBlock

(Pierce), 1 % BSA, 0.05 % Tween 20, 1 mM DTT). Inhibitors were kept at 40x stocks in 100% DMSO and added to the corresponding 384-well plates (40 μL). After 1 hour of incubation at 25 °C while shacking, affinity beads were washed with 0.05% Tween 20 in PBS. The beads were re-suspended in PBS buffer containing 0.05 % Tween 20 and 0.5 μM non-biotinylated affinity ligand, to then be incubated at 25 °C for 30 minutes while shacking. The kinase concentration present in the eluate was determined by qPCR. For further details, see Fabian, et. al., Nat. Biotechnol., 2005, 23 (3), 329-33.

NCI 60 cancer cell line screen: This screen was performed as per the description provided at http://dtp.nci.nih.gov/branches/btb/ivclsp.html. The NCI 60 panel cell lines were grown in RPMI 1640 medium supplemented with 5% (v/v) fetal bovine serum and 2mM L-glutamine at 37° C, 5 % CO2, 95 % air and 100 % relative humidity. Cells were seeded at 5,000 - 40,000 cells/well depending on their doubling time. After 24 hours of incubation, DMSO stock solutions of the compounds (400X) were added at 5 different concentrations (in 4, 10 or ½ log serial dilutions). After 48 h cells were fixed with trichloroacetic acid (10% or 16% final concentration for adherent cells and suspension cells respectively) and incubated for 60 minutes at 4°C. After discarding the supernatant, plates were washed with water (5x) and air dried. Sulforhodamine B (SRB) solution (100 μl) at 0.4 % (w/v) in 1% acetic acid was added to each well and incubated for 10 minutes at 4°C, followed by washing with 1% acetic acid (5x) and air drying. The bound stain was solubilized with 10mM trizma base and determined by absorbance measurement on an automated plate reader at a wavelength of 515 nm. The percentage growth inhibition was calculated as followed, being Tz time zero measurements, C control growth measurements and Ti test growth experiments in the present of the drug (see Figure 5 and 6).

$$[(Ti-Tz)/(C-Tz)] \times 100 \text{ for concentrations for which } Ti>/=Tz$$

$$[(Ti-Tz)/Tz] \times 100 \text{ for concentrations for which } Ti<Tz.$$

Three dose response parameters were calculated for each cell line. Growth inhibition of 50% (GI50) is calculated from [(Ti-Tz)/(C-Tz)] x 100 = 50 and corresponds to the drug concentration required in order to reduce by 50% the net protein increase in comparison to the control experiments. Total growth inhibition (TGI) is calculated from Ti = Tz. The LC50 indicates the drug concentration needed to reduce by 50% the measured protein at the end of the drug treatment as compared to that of the beginning and is derived from [(Ti-Tz)/Tz] x 100 = -50." Figures 5 and 6 provide the log base-10 values of GI50, TGI and LC50 of compound 10n and compound 100, respectively, as well as a graphical representation of the Z-score of each given value (GI50, TGI, LC50) for a given cell line relative to the mean and standard deviation of that value for all 60 cell lines.

## Claims

1. A compound **characterized by** a general formula (1),

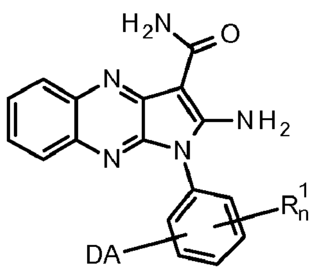

(1)

wherein n of $R^1_n$ is 0, 1, 2, 3 or 4, in particular n of $R^1$ is 0 or 1, and

- each $R^1$ independently from any other $R^1$ is $C_1$-$C_3$ alkyl, $C_1$-$C_3$ haloalkyl or $C_1$-$C_3$ alkoxy, in particular each $R^1$ independently from any other $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F, and

DA is $-C(=O)NH_2$, $-NHC(=O)OH$, $-NHC(=O)H$, $-C(=O)OH$, $-NHC(=O)NH_2$, $-C(=O)NHR^2$, $-NHC(=O)R^2$,

-NHC(=O)NHR$^2$, -NHC(=O)NR$^2_2$, -C(=S)NH$_2$, - NHC(=S)OH, -NHC(=S)H, -C(=S)OH, -NHC(=S)NH$_2$, $C_1$-$C_3$ thiol, C(=S)NHR$^2$, - NHC(=S)R$^2$, -NHC(=S)NHR$^2$, -NHC(=S)NR$^2_2$, a substituted or unsubstituted $C_5$-$C_6$ heteroaryl or a substituted or unsubstituted $C_5$-$C_6$ saturated heterocycle, and

- $R^2$ is a substituted or unsubstituted $C_3$-$C_8$ cycloalkyl, a substituted or unsubstituted $C_3$-$C_8$ halo cycloalkyl, a substituted or unsubstituted $C_3$-$C_8$ saturated heterocycle, a substituted or unsubstituted $C_3$-$C_8$ heteroaryl, a $C_1$-$C_6$ alkyl, a $C_1$-$C_6$ alkoxy or a $C_1$-$C_6$ haloalkyl, or
- $R^2$ is a substituent of the formula A

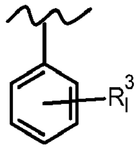

(formula A)

- with l of $R^3_l$ being 0, 1, 2, 3, 4 or 5, in particular l of $R^3_l$ being 0, 1 or 2, and
- each $R^3$ independently from any other $R^3$ is a $C_1$-$C_4$ alkyl, a $C_1$-$C_4$ alkoxy or a $C_1$-$C_4$ haloalkyl, a substituted or unsubstituted $C_5$-$C_6$ heteroaryl, in particular $R^3$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl F or a substituted or unsubstituted $C_5$-$C_6$ heteroaryl.

**2.** The compound according to claim 1, wherein

- n of $R^1_n$ is 0 or 1, and
- $R^1$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl or F in particular $R^1$ is $CH_3$, Cl or F.

**3.** The compound according to any one of claim 1 or 2, wherein DA is -C(=O)NHR$^2$, -NHC(=O)R$^2$, -NHC(=O)NHR$^2$ -NHC(=S)NHR$^2$, C(=O)NR$^2_2$ or -NHC(=O)NR$^2_2$, in particular DA is -C(=O)NHR$^2$, -NHC(=O)R$^2$, -NHC(=O)NHR$^2$ or- NHC(=S)NHR$^2$ and

- $R^2$ is a substituted or unsubstituted $C_5$-$C_8$ cycloalkyl, a substituted or unsubstituted $C_5$-$C_8$ halocycloalkyl, a substituted or unsubstituted $C_5$-$C_8$ saturated heterocycle, in particular a substituted or unsubstituted $C_5$-$C_6$ saturated heterocycle, a substituted or unsubstituted $C_3$-$C_8$ heteroaryl, in particular a substituted or unsubstituted $C_5$-$C_6$ heteroaryl.

**4.** The compound according to any one of claim 1, 2 or 3, wherein

- $R^2$ is a substituted or unsubstituted $C_5$-$C_6$ saturated heterocycle or a substituted or unsubstituted $C_5$-$C_6$ heteroaryl, in particular a substituted or unsubstituted imidazole or a substituted or unsubstituted pyrazole.

**5.** The compound according to any one of claim 1 or 2, wherein DA is -C(=O)NHR$^2$, -NHC(=O)R$^2$, -NHC(=O)NHR$^2$ -NHC(=S)NHR$^2$, C(=O)NR$^2_2$, -NHC(=O)NR$^2_2$, in particular DA is -C(=O)NHR$^2$, -NHC(=O)R$^2$, -NHC(=O)NHR$^2$ or-NHC(=S)NHR$^2$, and $R^2$ is a substituent of the formula A,

- with l of $R^3_l$ being 0, 1, 2, 3, 4 or 5, in particular l of $R^3_l$ being 0, 1 or 2, and
- each $R^3$ independently from any other $R^3$ is a $C_1$-$C_4$ alkyl, a $C_1$-$C_4$ alkoxy or a $C_1$-$C_4$ haloalkyl, a substituted or unsubstituted $C_5$-$C_6$ heteroaryl, in particular $R^3$ is $CH_3$, $OCH_3$, $CF_3$, $CHF_2$, $CH_2F$, $CH_2CF_3$, $CH_2CHF_2$, $CH_2CH_2F$, $CHFCF_3$, $CHFCHF_2$, $CHFCH_2F$, $CF_2CF_3$, $CF_2CHF_2$, $CF_2CH_2F$, Cl, F or a substituted or unsubstituted $C_5$-$C_6$ heteroaryl.

**6.** The compound according to any one of claims 1, 2 or 5, wherein $R^2$ is a substituent of the formula A

- with l of $R^3_l$ being 1 or 2, and
- each $R^3$ independently from any other $R^3$ is a substituted or unsubstituted $C_5$-$C_6$ heteroaryl, in particular a substituted or unsubstituted imidazole, $CH_3$, $CH_2F$, $CHF_2$, $CF_3$, $CH_2CF_3$, $CH_2CHF_2$, $CH_2CH_2F$, $OCH_3$, Cl or F,

wherein in particular each $R^3$ is in meta position in relation to the attachment position of the benzene moiety of $R^3$ to DA, and wherein in particular each $R^3$ independently from any other $R^3$ is $CF_3$ or a substituted or unsubstituted imidazole connected to the benzene moiety of $R^2$ in position 1, in particular a substituted imidazole comprising a $C_1$-$C_6$ alkyl, in particular a $C_1$-alkyl, in position 4.

**7.** The compound according to any one of claim 1 or 2, wherein
DA is -C(=O)NH$R^2$, -NHC(=O)$R^2$, -NHC(=O)NH$R^2$, -NHC(=S)NH$R^2$, C(=O)N$R^2_2$ or - NHC(=O)N$R^2_2$, in particular DA is -C(=O)NH$R^2$, -NHC(=O)$R^2$, -NHC(=O)NHR or- NHC(=S)NH$R^2$, and

- $R^2$ is a substituted or unsubstituted $C_5$-$C_6$ heteroaryl, in particular a substituted or unsubstituted $C_5$ heteroaryl, more particularly substituted or unsubstituted imidazole or a substituted or unsubstituted pyrazole.

**8.** The compound according to any one of the claims 1, 2 or 7, wherein

- $R^2$ is a substituted or unsubstituted pyrazole, in particular a pyrazole connected to the benzene moiety of $R^2$ in position 5.

**9.** The compound according to any one of the claims 7 to 8, wherein said pyrazole is a substituted pyrazole comprising

- a $C_1$-$C_6$ alkyl in position 3, in particular t-butyl in position 3, and/or
- a substituted or unsubstituted $C_6$ to $C_{14}$ aryl or heteroaryl group, wherein in particular said $C_6$ to $C_{14}$ aryl or heteroaryl group is connected to said pyrazole in position 1.

**10.** The compound according to claim 9, wherein said $C_6$ to $C_{14}$ aryl or heteroaryl group is

- a substituted or unsubstituted quinoline, in particular a unsubstituted quinoline, or
- a substituted or unsubstituted phenyl group, in particular a substituted phenyl group comprising a $C_1$-$C_6$ alkyl, in particular $CH_3$, wherein in particular said $C_1$-$C_6$ alkyl is situated in para position in relation to the attachment position of the phenyl group to said pyrazole.

**11.** The compound according to any one of the previous claims, wherein

- n of $R^1_n$ is 1 and $R^1$ is situated in ortho position in relation to the attachment position of the benzene moiety to the parent moiety, and/or
- DA is in meta or para position in relation to the attachment position of the benzene moiety to the parent moiety, or
- n of $R^1_n$ is 1 and $R^1$ is situated in ortho and DA is in meta position in relation to the attachment position of the benzene moiety to the parent moiety yielding a 1, 2, 5 substitution pattern.

**12.** The compound according to any one of the previous claims, wherein

- DA is -C(=O)NH$R^2$, -NHC(=O)$R^2$ or C(=O)N$R^2_2$ and DA is in meta position in relation to the attachment position of the benzene moiety; or
- DA is -NHC(=O)NH$R^2$ or -NHC(=S)NH$R^2$ and DA is in para position in relation to the attachment position of the benzene moiety.

**13.** A pharmaceutical preparation comprising at least one compound according to one of the claims 1 to 12 as an active ingredient.

**14.** A compound according to any of the claims 1 to 12 or a pharmaceutical preparation according to claim 13 for use in a method of treatment of disease.

**15.** A compound according to any of the claims 1 to 12 or a pharmaceutical preparation according to claim 13 for use in a method for treatment of cancer or intraocular neovascular syndromes, in particular for use in a method for treatment of cancer.

Fig. 1

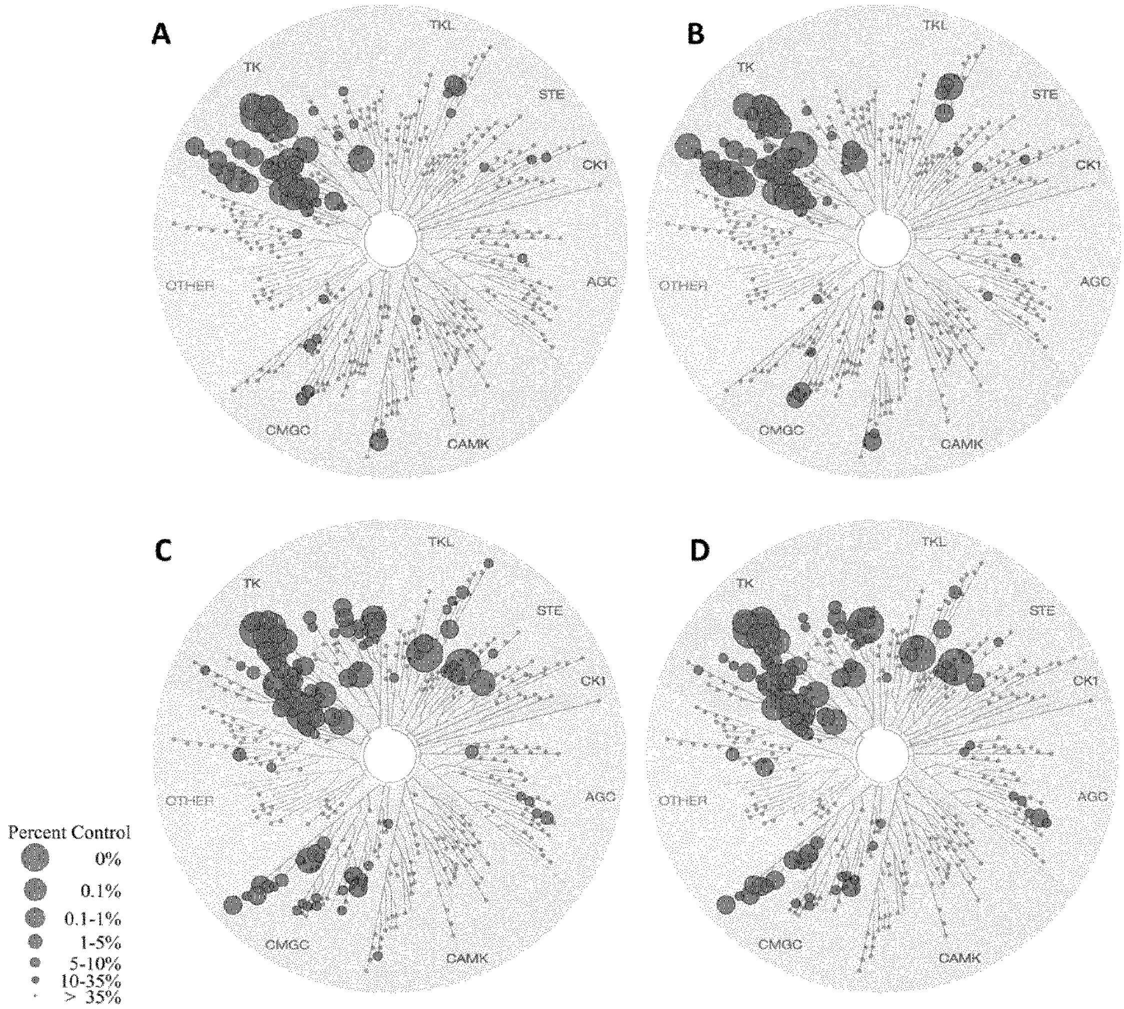
A
B
C
D
TK
TKL
STE
CK1
AGC
CAMK
CMGC
OTHER
Percent Control
0%
0.1%
0.1-1%
1-5%
5-10%
10-35%
> 35%

Fig. 2

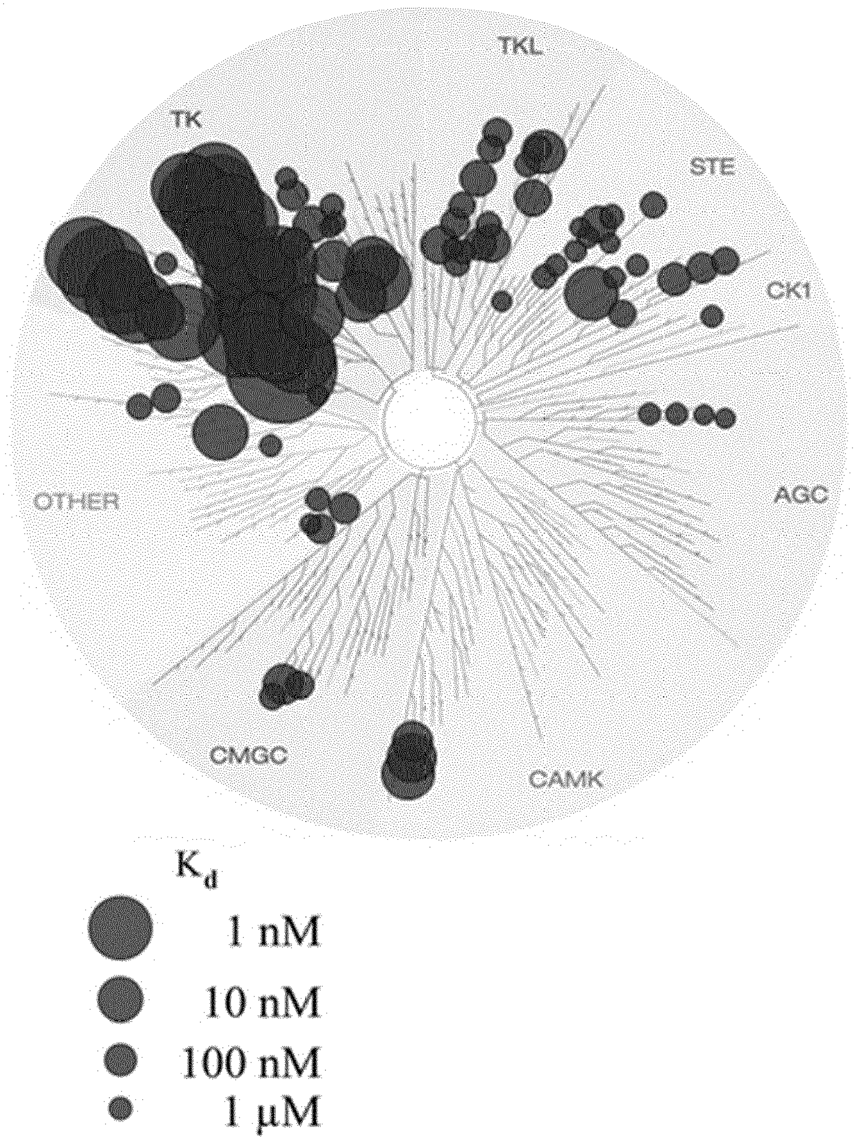

Fig. 3

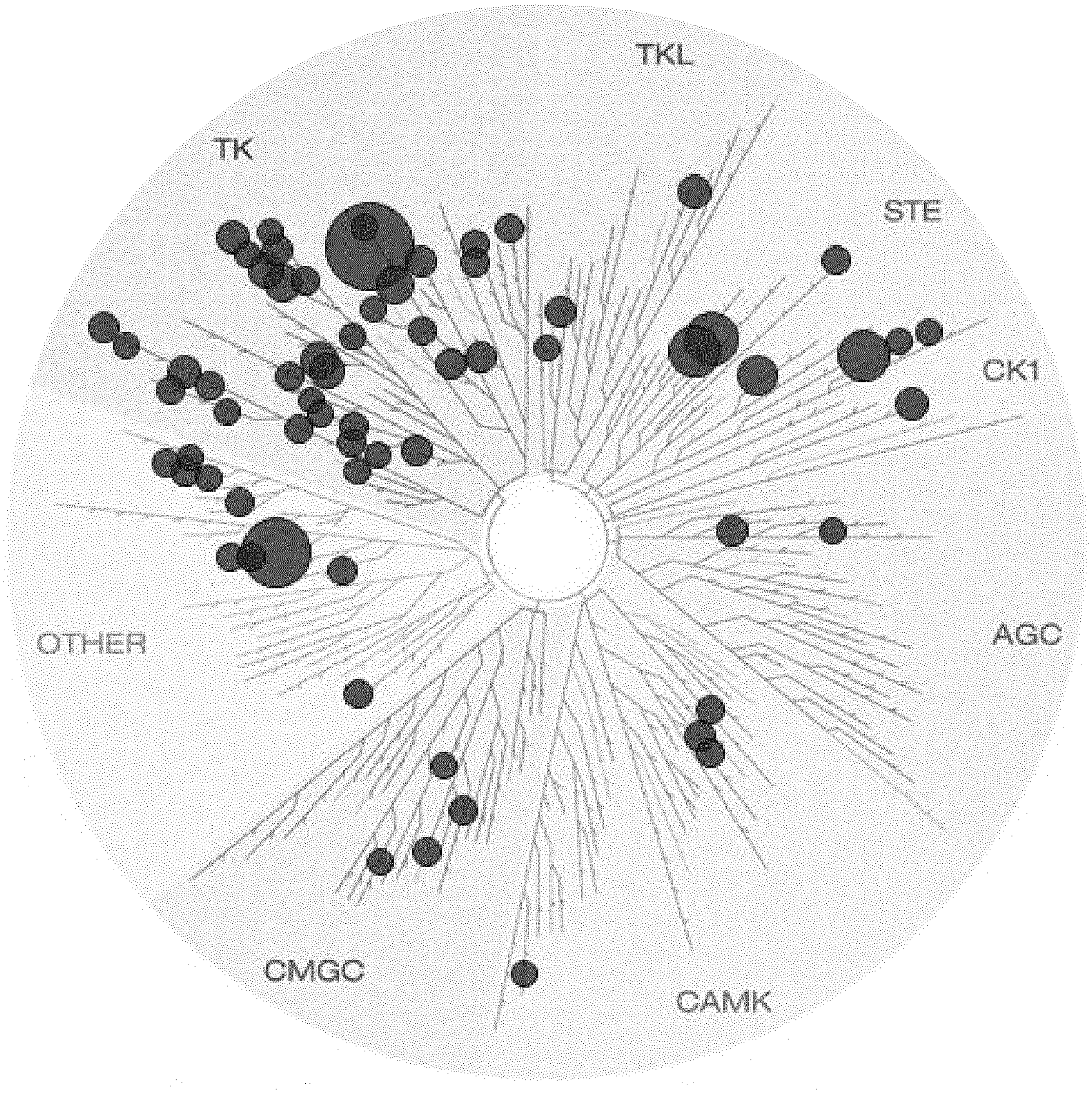

Fig. 4

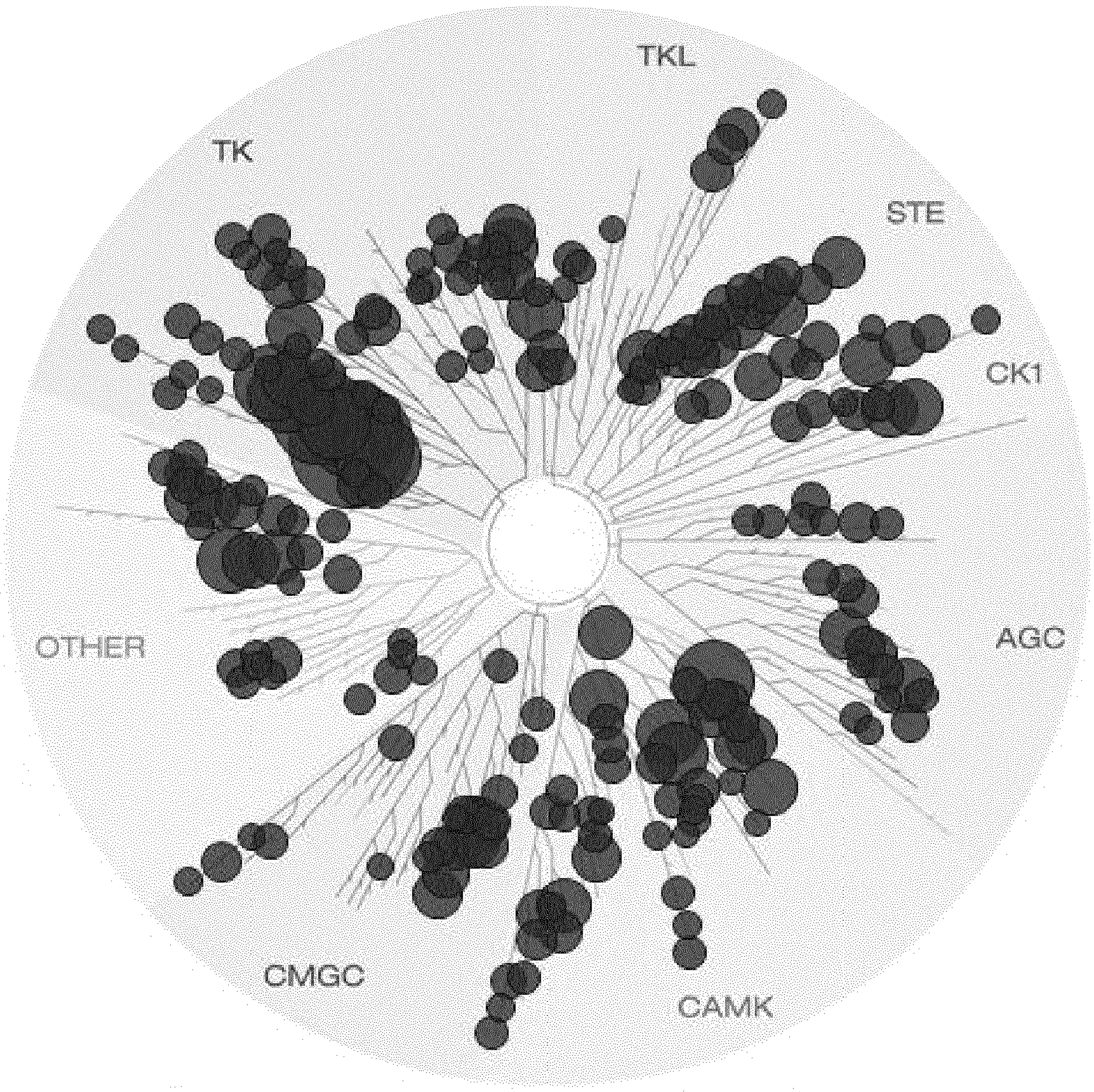

Fig. 5

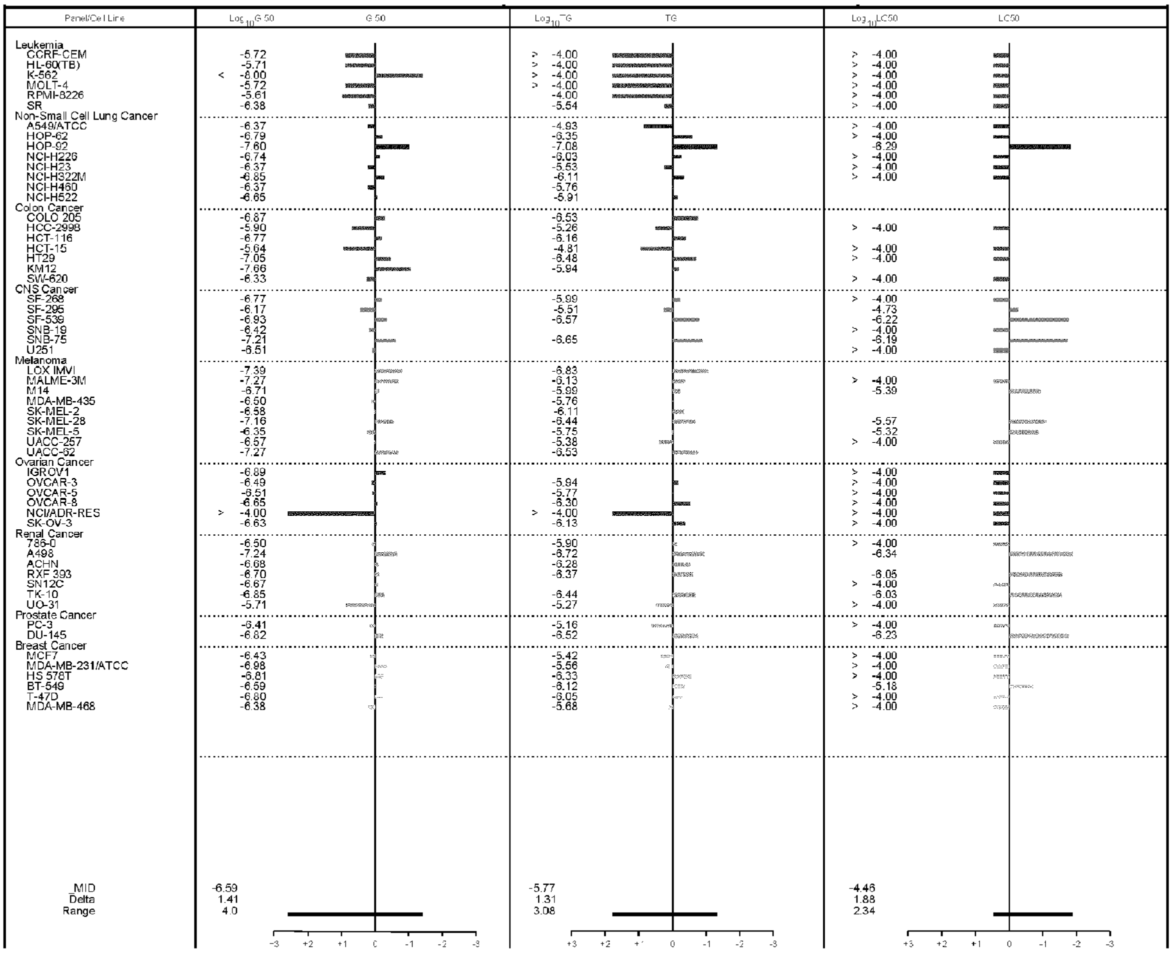
Panel/Cell Line
Log10GI50
GI50
Log10TGI
TGI
Log10LC50
LC50
Leukemia
CCRF-CEM -5.72 > -4.00 > -4.00
HL-60(TB) -5.71 > -4.00 > -4.00
K-562 < -8.00 > -4.00 > -4.00
MOLT-4 -5.72 > -4.00 > -4.00
RPMI-8226 -5.61 -4.00 > -4.00
SR -6.38 -5.54 > -4.00
Non-Small Cell Lung Cancer
A549/ATCC -6.37 -4.93 > -4.00
HOP-62 -6.79 -6.35 > -4.00
HOP-92 -7.60 -7.08 -6.29
NCI-H226 -6.74 -6.03 > -4.00
NCI-H23 -6.37 -5.53 > -4.00
NCI-H322M -6.85 -6.11 > -4.00
NCI-H460 -6.37 -5.76
NCI-H522 -6.65 -5.91
Colon Cancer
COLO 205 -6.87 -6.53
HCC-2998 -5.90 -5.26 > -4.00
HCT-116 -6.77 -6.16
HCT-15 -5.64 -4.81 > -4.00
HT29 -7.05 -6.48 > -4.00
KM12 -7.66 -5.94
SW-620 -6.33 > -4.00
CNS Cancer
SF-268 -6.77 -5.99 > -4.00
SF-295 -6.17 -5.51 -4.73
SF-539 -6.93 -6.57 -6.22
SNB-19 -6.42 > -4.00
SNB-75 -7.21 -6.65 -6.19
U251 -6.51 > -4.00
Melanoma
LOX IMVI -7.39 -6.83
MALME-3M -7.27 -6.13 > -4.00
M14 -6.71 -5.99 -5.39
MDA-MB-435 -6.50 -5.76
SK-MEL-2 -6.58 -6.11
SK-MEL-28 -7.16 -6.44 -5.57
SK-MEL-5 -6.35 -5.75 -5.32
UACC-257 -6.57 -5.38 > -4.00
UACC-62 -7.27 -6.53
Ovarian Cancer
IGROV1 -6.89 > -4.00
OVCAR-3 -6.49 -5.94 > -4.00
OVCAR-5 -6.51 -5.77 > -4.00
OVCAR-8 -6.65 -6.30 > -4.00
NCI/ADR-RES > -4.00 > -4.00 > -4.00
SK-OV-3 -6.63 -6.13 > -4.00
Renal Cancer
786-0 -6.50 -5.90 > -4.00
A498 -7.24 -6.72 -6.34
ACHN -6.68 -6.28
RXF 393 -6.70 -6.37 -6.05
SN12C -6.67 > -4.00
TK-10 -6.85 -6.44 -6.03
UO-31 -5.71 -5.27 > -4.00
Prostate Cancer
PC-3 -6.41 -5.16 > -4.00
DU-145 -6.82 -6.52 -6.23
Breast Cancer
MCF7 -6.43 -5.42 > -4.00
MDA-MB-231/ATCC -6.98 -5.56 > -4.00
HS 578T -6.81 -6.33 > -4.00
BT-549 -6.59 -6.12 -5.18
T-47D -6.80 -6.05 > -4.00
MDA-MB-468 -6.38 -5.68 > -4.00
MID -6.59 -5.77 -4.46
Delta 1.41 1.31 1.88
Range 4.0 3.08 2.34
+3 +2 +1 0 -1 -2 -3

Fig. 6

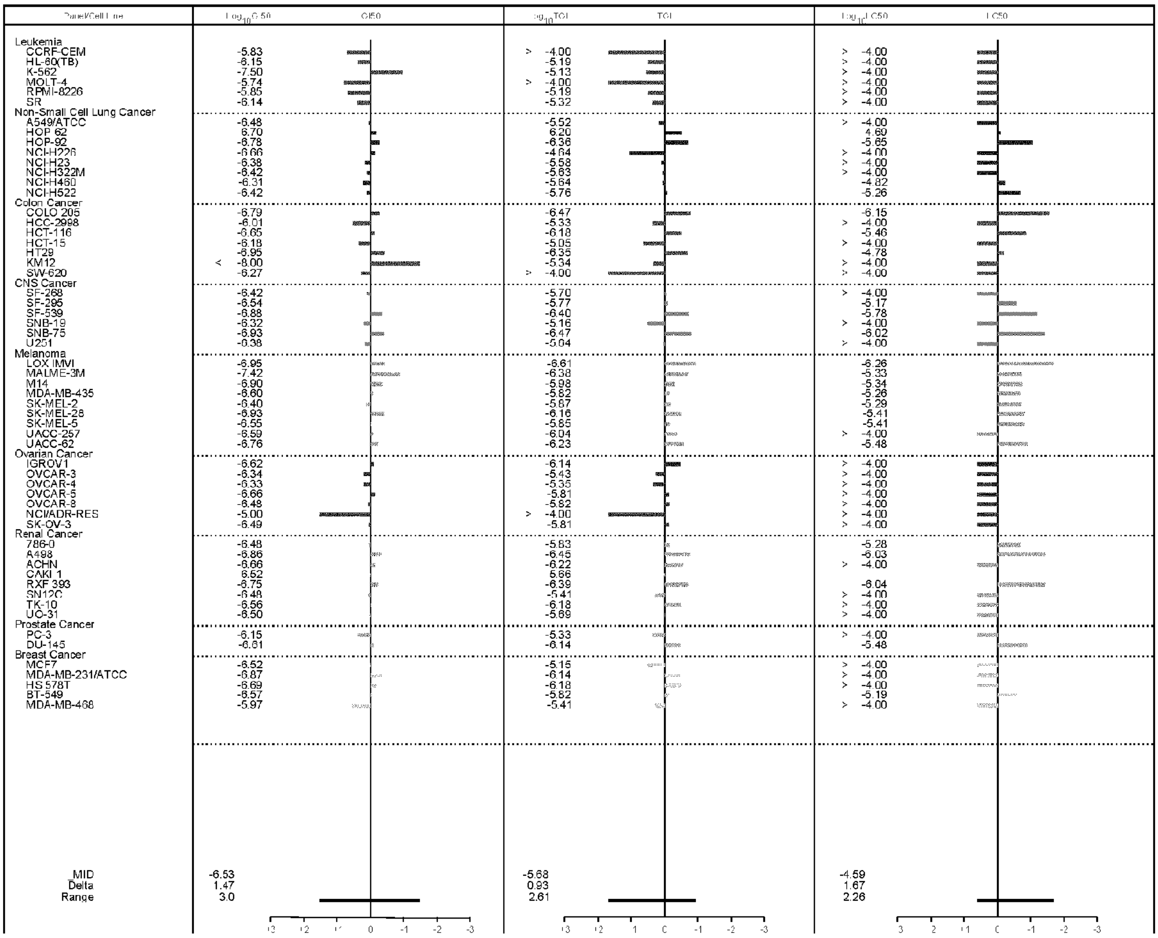
Panel/Cell Line
Log10 GI50
GI50
Log10 TGI
TGI
Log10 LC50
LC50
Leukemia
CCRF-CEM -5.83 > -4.00 > -4.00
HL-60(TB) -6.15 -5.19 > -4.00
K-562 -7.50 -5.13 > -4.00
MOLT-4 -5.74 > -4.00 > -4.00
RPMI-8226 -5.85 -5.19 > -4.00
SR -6.14 -5.32 > -4.00
Non-Small Cell Lung Cancer
A549/ATCC -6.48 -5.52 > -4.00
HOP 62 6.70 6.20 4.69
HOP-92 -6.78 -6.36 -5.65
NCI-H226 -6.66 -4.64 > -4.00
NCI-H23 -6.38 -5.58 > -4.00
NCI-H322M -6.42 -5.63 > -4.00
NCI-H460 -6.31 -5.64 -4.82
NCI-H522 -6.42 -5.76 -5.26
Colon Cancer
COLO 205 -6.79 -6.47 -6.15
HCC-2998 -6.01 -5.33 > -4.00
HCT-116 -6.65 -6.18 -5.46
HCT-15 -6.18 -5.05 > -4.00
HT29 -6.95 -6.35 -4.78
KM12 < -8.00 -5.34 > -4.00
SW-620 -6.27 > -4.00 > -4.00
CNS Cancer
SF-268 -6.42 -5.70 > -4.00
SF-295 -6.54 -5.77 -5.17
SF-539 -6.88 -6.40 -5.78
SNB-19 -6.32 -5.16 > -4.00
SNB-75 -6.93 -6.47 -6.02
U251 -6.38 -5.04 > -4.00
Melanoma
LOX IMVI -6.95 -6.61 -6.26
MALME-3M -7.42 -6.38 -5.33
M14 -6.90 -5.98 -5.34
MDA-MB-435 -6.60 -5.82 -5.26
SK-MEL-2 -6.40 -5.87 -5.29
SK-MEL-28 -6.93 -6.16 -5.41
SK-MEL-5 -6.55 -5.85 -5.41
UACC-257 -6.59 -6.04 > -4.00
UACC-62 -6.76 -6.23 -5.48
Ovarian Cancer
IGROV1 -6.62 -6.14 > -4.00
OVCAR-3 -6.34 -5.43 > -4.00
OVCAR-4 -6.33 -5.35 > -4.00
OVCAR-5 -6.66 -5.81 > -4.00
OVCAR-8 -6.48 -5.82 > -4.00
NCI/ADR-RES -5.00 > -4.00 > -4.00
SK-OV-3 -6.49 -5.81 > -4.00
Renal Cancer
786-0 -6.48 -5.83 -5.28
A498 -6.86 -6.45 -6.03
ACHN -6.66 -6.22 > -4.00
CAKI 1 6.52 5.66
RXF 393 -6.75 -6.39 -6.04
SN12C -6.48 -5.41 > -4.00
TK-10 -6.56 -6.18 > -4.00
UO-31 -6.50 -5.69 > -4.00
Prostate Cancer
PC-3 -6.15 -5.33 > -4.00
DU-145 -6.61 -6.14 -5.48
Breast Cancer
MCF7 -6.52 -5.15 > -4.00
MDA-MB-231/ATCC -6.87 -6.14 > -4.00
HS 578T -6.69 -6.18 > -4.00
BT-549 -6.57 -5.82 -5.19
MDA-MB-468 -5.97 -5.41 > -4.00
MID -6.53 -5.68 -4.59
Delta 1.47 0.93 1.67
Range 3.0 2.61 2.26

Europäisches Patentamt
European Patent Office
Office européen des brevets

# EUROPEAN SEARCH REPORT

Application Number
EP 14 16 2145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | HONGTAO ZHAO ET AL: "Hydrogen Bonding Penalty upon Ligand Binding", PLOS ONE, vol. 6, no. 6, 17 June 2011 (2011-06-17), page e19923, XP055130798, ISSN: 1932-6203, DOI: 10.1371/journal.pone.0019923 * abstract * * page 6; figure 5 * * page 6, left-hand column, last paragraph * ----- | 1-15 | INV. C07D487/04 A61K31/497 A61P35/00 |
| A,D | LAFLEUR ET AL: "Design and synthesis of selective kinase inhibitors", 23 January 2012 (2012-01-23), DISSERTATION,, PAGE(S) 166PP, XP009179379, * page 121 - page 136; table 2; compounds 10-15 * ----- | 1-15 | |
| A | MYUNG-HO JUNG ET AL: "Design, synthesis, and antiproliferative activity of new 1-pyrrolo[3,2-]pyridine derivatives against melanoma cell lines. Part 2", BIOORGANIC & MEDICINAL CHEMISTRY LETTERS, PERGAMON, AMSTERDAM, NL, vol. 22, no. 13, 9 May 2012 (2012-05-09), pages 4362-4367, XP028491010, ISSN: 0960-894X, DOI: 10.1016/J.BMCL.2012.05.004 [retrieved on 2012-05-09] * abstract * * Figure 1, Schemes 1 and 2 and Tables 1 and 2 * ----- | 1-15 | TECHNICAL FIELDS SEARCHED (IPC) C07D A61K A61P |

The present search report has been drawn up for all claims

| Place of search | Date of completion of the search | Examiner |
|---|---|---|
| Munich | 31 July 2014 | Papathoma, Sofia |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006024834 A1, Aquila, B.
- WO 2007076034 A2, Betebenner, D.A.


### Non-patent literature cited in the description


- **CHENG N. ; BRANTLEY D.M. ; CHEN J.** *Cytokine Growth Factor Rev.,* 2002, vol. 13, 75-85
- **MIYAZAKI Y. et al.** *Bioorg Med Chem Lett,* 2007, vol. 17, 250-254
- **ZHAO H. ; HUANG D.** *PLoS ONE,* 2011, vol. 6 (6
- **MARTINY-BARON G. et al.** *Angiogenesis,* 2010, vol. 13 (3), 259-67
- **FOLKMAN et al.** *J. Biol. Chem.,* 1992, vol. 267, 10931-34
- **KLAGSBRUN et al.** *Annu. Rev. Physiol.,* 1991, vol. 53, 217-39
- Vascular diseases. **GARNER A.** Pathobiology of Ocular Disease. A Dynamic Approach. Marcel Dekker, 1994, 1625-1710
- **PRATT, E.F. et al.** *J Org Chem,* 1967, vol. 32 (1), 49-53
- **OBAFEMI, C.A. et al.** *Molecules,* 2004, vol. 9 (4), 223-231
- **DEAK, H.L. et al.** *Bioorg Med Chem Lett,* 2008, vol. 18 (3), 1172-1176
- **OGURO, Y. et al.** *Bioorgan Med Chem,* 2010, vol. 18 (20), 7260-7273
- **CHOI, H.G. et al.** *J Med Chem,* 2010, vol. 53 (15), 5439-5448
- **ZHAO H. ; HUANG D.** Hydrogen Bonding Penalty upon Ligand Binding. *PLoS ONE,* 2011, vol. 6 (6
- **JECKLIN M.C. et al.** *Journal of Molecular Recognition,* vol. 22 (4), 319-329
- **LAFLEUR K ; DONG J. ; HUANG D. ; CAFLISCH A. ; C. NEVADO.** Experimental Section. *Journal of Medicinal Chemistry,* 2013, vol. 56 (1), 84-96
- **CHOI, Y. et al.** *Bioorg Med Chem Lett,* 2009, vol. 19 (15), 4467-4470
- **FABIAN.** *Nat. Biotechnol.,* 2005, vol. 23 (3), 329-33